# EUROPEAN PATENT APPLICATION

(11) **EP 3 977 948 A1**
(43) Date of publication of application: **06.04.2022**
(21) Application number: 20797987.3
(22) Date of filing: 01.05.2020
(51) Int. Cl.: A61B 17/34

(54) **PUNCTURE CORE ASSEMBLY AND PUNCTURE DEVICE HAVING PUNCTURE CORE ASSEMBLY**

(30) Priority: 02.05.2019 CN 201910367365
(71) Applicant: Fulbright Medical Inc., Wuxi, Jiangsu 214437 (CN)
(72) Inventor: SUN, Baofeng, Wuxi, Jiangsu 214437 (CN); HUANG, Ye, Wuxi, Jiangsu 214437 (CN)
(74) Representative: Novagraaf Technologies
(86) International application number: PCT/CN2020/088551
(87) International publication number: WO 2020/221369

(57) **Abstract**

Provided in the present invention is a puncture core assembly, comprising an operating assembly, a puncture core rod, and a puncture tip used for puncturing human tissue so as to form a puncture opening. The operating assembly is manipulated to generate a driving force. The puncture core rod comprises a transmission assembly and an execution assembly. The transmission assembly transmits the driving force. The execution assembly has a suture actuator, and also has a positioning assembly, a suture assembly, a suture thread release assembly, and a suture needle fixing assembly, which are used to perform positioning when suturing the puncture opening, suture out a needle, release a suture thread, and receive and hold the suture needle respectively, so that the puncture device provided with the foregoing puncture core assembly becomes a puncture device that is capable of suturing, provided with complete functions, and occupies less layout space.

## Description

### Technical Field

The present invention relates to a surgical instrument, in particular relates to a puncture core assembly and a puncture device, and belongs to a field of medical equipment.

### Background

In minimally invasive surgery such as abdominal surgery and thoracic surgery, a puncture device establishes an access channel in a cavity wall of a human body, so that a stapler or other surgical instruments (such as an endoscope) enter a body cavity, and provide a gas access channel for inspection or surgical operation.

The puncture device includes a puncture core assembly and a cannula assembly. During an operation, a doctor usually cuts a small incision on a human tissue of a patient first, then aligns a puncture tip of the puncture core assembly with the small incision, performs left and right rotation in a reciprocating manner, and downwardly moves the puncture device at the same time, so that the puncture core assembly guides the cannula assembly to penetrate through a cortex of the human tissue of the patient, then the doctor pulls out the puncture core assembly, and the stapler or other surgical instruments enters and exits a body cavity of the patient through the cannula assembly for operation. An existing puncture core assembly only plays a role of puncturing, and is discarded after guiding the cannula assembly from an incision of a human abdomen into the body of the patient.

At the end of the operation, the cannula assembly is taken out from a puncture opening, and the puncture opening is sutured. Because the puncture opening of minimally invasive surgery is small and deep, especially a human tissue of an obese patient is relatively thick, if the suture is improper, the patient is prone to complications such as puncture hole hernia after operation. A special suture instrument is used to suture the puncture opening to reduce the above complications, but this requires additional surgical instruments, relatively high suture cost, a large number of surgical instruments and inconvenient operation. Moreover, a suture device specially suturing the puncture opening is complex in structure and inconvenient to use. Therefore, the puncture device with a suture function is used.

In order to achieve accurate suture, the puncture device with the suture function should position the human tissue on both sides of the puncture opening at first before suture. Therefore, the puncture device needs to be equipped with a device that can realize positioning. However, an existing suturable puncture device has inaccurate positioning and inconvenient operation of a positioning device, and how to layout a device driving a positioning action and a device performing the positioning action within a limited containing space of the puncture device has become a technical problem to be solved by those skilled in the art.

After the positioning action is performed, a needle withdrawing action is performed to suture the puncture opening. In an actual operation, there are two suture modes for the puncture opening. The first suture mode is: a suture needle drives a suture thread to enter the puncture opening from the outside of the puncture opening, then penetrate in from a side surface of the puncture opening, and penetrate out from the human tissue on both sides of the puncture opening. Using this suture mode, it is not necessary to send the suture thread into a body in advance, however, when the suture needle penetrates in from the side surface of the puncture opening, it will exert thrust on the side surface of the puncture opening, so that the puncture opening is expanded, the suture effect is not ideal, and a fascia layer can not be sutured. The second suture mode is: the puncture opening is sutured from inside to outside or from outside to inside, and the suture trajectory is a straight line. This suture mode can not achieve accurate positioning or clamping on the tissue, which is easy to lead to suture failure. The following suture mode is relatively ideal: the suture needle drives the suture thread to penetrate in from the lowest layer of tissue on both sides of the puncture opening (i.e. the fascia layer) and out from the side surface of the puncture opening, so that the fascia layer can be well sutured. However, the realization of this suture mode requires a corresponding suture driving device and suture actuator, and the suture thread needs to be sent to the body before suture. What kind of structures do the suture driving device and the suture actuator adopt to realize the corresponding functions has become a technical problem to be solved. It is a feasible way to put the suture thread in the puncture core assembly and make the suture thread follow the puncture core assembly into the puncture opening. However, how to put the suture thread in the puncture core assembly and how to release the suture thread and cooperate with suturing out of a needle become a technical problem to be solved when the above suture mode is used. In addition, in a limited containing space of the puncture device, how to layout the suture driving device, the suture actuator, a driving device and an executing device for releasing the suture thread also become a technical problem that needs to be further solved by those skilled in the art.

In some existing puncture devices that are capable of suturing, a suture assembly for suture includes a suture arm and a suture needle located at the end of the suture arm, and the suture needle is tied with the suture thread. After suture, the suture needle needs to be separated from the suture arm to leave the suture thread of the suture needle. However, the suture needle and the suture arm need a large external force to separate them. A needle receiving assembly used to leave the suture needle in the traditional art has poor fixation effect on the suture needle, and the suture needle can not be effectively separated from the suture arm. Therefore, how to improve the fixation effect of the suture needle to reduce the probability of separation failure between the suture arm and the suture needle has become a technical problem to be solved by those skilled in the art.

An existing suturable puncture device does not have a device to prevent misoperation. In addition, the arrangement of a device with a function of avoiding misoperation by a doctor will occupy the containing space of the puncture device, and the containing space of the puncture device is very limited.

### Summary

In order to solve the above problems, some embodiments of the present invention provide a positioning mechanism for a puncture core assembly.

The positioning mechanism for the puncture core assembly includes a positioning operating assembly and a positioning assembly. The positioning assembly is driven by the positioning operating assembly to rotate with a first axial direction of the puncture core assembly as a central axis.

In some embodiments, the positioning operating assembly includes a toggle member, which is applied with a force along a circumferential direction of the puncture core assembly, so as to drive the positioning operating assembly to rotate with a second axial direction of the puncture core assembly as a central axis, so as to drive the positioning assembly. The first axial direction and the second axial direction are parallel and not coaxial.

In some embodiments, the second axial direction is a central axis of the puncture core assembly.

In some embodiments, the positioning mechanism also includes a positioning transmission assembly, a near end of the positioning transmission assembly is fixedly connected with the toggle member, and a far end of the positioning transmission assembly is drivably connected with the near end of the positioning assembly.

In some embodiments, the far end of the positioning transmission assembly includes a protruding part, and the near end of the positioning assembly includes a containing groove. The protruding part is movably contained in the containing groove to form the driveable connection.

In some embodiments, the containing groove is a waist-shaped groove.

In some embodiments, the positioning assembly includes a positioning blade. After the positioning blade rotates, a suture channel of the puncture core assembly is exposed.

In some embodiments, the far end of the positioning transmission assembly is a rotating ring, and the protruding part is formed by protruding from the upper surface or lower surface of the rotating ring.

In some embodiments, the positioning assembly includes a movement aiding arm, a pivot shaft and a positioning blade, the movement aiding arm extends horizontally from the near end of the pivot shaft, the movement aiding arm includes the containing groove, and the positioning blade is located at the far end of the pivot shaft.

In some embodiments, the puncture core assembly also includes a suture mechanism and a suture thread release mechanism.

In some embodiments, the positioning assembly has a closed state and an open state. The conversion between the closed state and the open state is realized by the rotation of the positioning assembly with the first axial direction as the central axis.

In some embodiments, the positioning assembly includes a positioning blade. In the closed state, the positioning blade is flush with the outer surface of the puncture core assembly. In the open state, the positioning blade protrudes out of the outer surface of the puncture core assembly.

In some embodiments, the positioning blade has an upper surface which is perpendicular to the central axis of the puncture core assembly. In the open state, the upper surface of the positioning blade is in surface contact with tissue.

Some embodiments of the present invention provide a suture thread release mechanism for the puncture core assembly, the suture thread release mechanism includes a first wall housing and a second wall housing. The suture thread release mechanism has an initial state and a termination state. In the initial state, the first wall housing is closed with the second wall housing. In the termination state, the first wall housing is separated from the second wall housing. When the first wall housing and the second wall housing are closed, they are surrounded by each other to form an inner cavity, which is used to contain the suture thread.

In some embodiments, the size of the first wall housing is different from that of the second wall housing.

In some embodiments, the first wall housing is pivoted relative to the second wall housing to realize the conversion between the initial state and the termination state.

In some embodiments, one of the first wall housing and the second wall housing is provided with a toothed member, or, both the first wall housing and the second wall housing are provided with teeth members.

In some embodiments, the suture thread release mechanism also includes a suture thread release operating assembly and a suture thread release transmission assembly, the suture thread release operating assembly is pressed or pulled to drive the suture thread release transmission assembly to move in a straight line, and the suture thread release transmission assembly drives the relative pivoting of the first wall housing and the second wall housing.

In some embodiments, the suture thread release operating assembly is butted against the suture thread release transmission assembly to drive the suture thread transmission assembly.

In some embodiments, the suture thread release transmission assembly includes a rack.

In some embodiments, the parts of the two opposite side surfaces of the rack are provided with teeth, and the parts of the two side surfaces are symmetrical to each other; alternatively, a part of a side surface of the rack is provided with teeth.

In some embodiments, in the initial state, the first wall housing and the second wall housing are closed to form a puncture tip, and the inner cavity is an inner cavity of the puncture tip.

In some embodiments, the puncture core assembly also includes a suture mechanism, the suture mechanism includes a suture operating assembly, a suture transmission assembly and a suture execution assembly, the suture operating assembly drives the suture execution assembly through the suture transmission assembly, the suture execution assembly includes a suture needle, the suture transmission assembly and the suture thread release transmission assembly both include a same rack.

Some embodiments of the present invention provide a suture needle fixing mechanism includes a movable member, the movable member includes a slit extending from the end of the movable member, and the slit is used to hold the suture needle.

In some embodiments, the suture needle fixing mechanism also includes a suture needle fixing transmission assembly, the movable member is fixedly connected with the suture needle fixing transmission assembly, and the suture needle fixing transmission assembly drives the movable member to move in a straight line.

In some embodiments, the suture needle fixing transmission assembly sequentially includes an upper transmission ring, connecting rods, a transmission pipe, a lower transmission ring and two booster arms from top to bottom, the two booster arms are symmetrical disposed. There are two connecting rods which are symmetrically disposed with each other. The upper transmission ring is fixedly connected with the near ends of the two connecting rods, and far ends of the two connecting rods are fixedly connected with the near end of the transmission pipe. A far end of the transmission pipe is fixedly connected with the lower transmission ring, the lower transmission ring is fixedly connected with the near ends of the two booster arms, there are two movable members, and the movable members are fixedly connected with the booster arms in one-to-one correspondence.

In some embodiments, the suture needle fixing mechanism also includes a suture needle fixing operating assembly, the suture needle fixing operating assembly is butted against the suture needle fixing transmission assembly after being pressed so as to drive the suture needle fixing transmission assembly to move in a straight line, and the suture needle fixing operating assembly is separated from the suture needle fixing transmission assembly after being pulled.

In some embodiments, the puncture core assembly also includes a suture mechanism and a suture thread release mechanism, the suture mechanism includes a suture operating assembly, the suture thread release mechanism includes a suture thread release operating assembly, and the suture needle fixing operating assembly, the suture operating assembly and the suture thread release operating assembly are the same operating assembly.

In some embodiments, the same operating assembly includes a pressing cover, which is pressed or pulled to move the same operating assembly downwardly or upwardly.

In some embodiments, the suture needle fixing mechanism also includes a blocking arm used to block the movable member holding the suture needle.

In some embodiments, the suture needle fixing mechanism also includes a receiving assembly, the receiving assembly includes a receiving sheet, and the receiving sheet is an elastic grid sheet or an elastic hollow sheet of the suture needle used for holding the suture needle.

Some embodiments of the present invention provide a suture mechanism for the puncture core assembly, the suture mechanism includes two suture members, each suture member includes a suture arm and a suture needle, the suture needle is separately connected to the suture arm, an end of a suture thread is fixed to the suture needle, and the two suture members are used for being driven to rotate to realize suturing out of a needle.

In some embodiments, each suture member also includes a gear, the suture mechanism also includes a suture transmission assembly, the suture transmission assembly includes a rack, and the gear is engaged with the rack.

In some embodiments, the suture mechanism also includes a suture operating assembly, and the suture operating assembly drives the suture transmission assembly to move in a straight line.

In some embodiments, the puncture core assembly also includes a suture thread release mechanism, and the suture thread release mechanism includes a suture thread release transmission assembly, the suture transmission assembly and the suture thread release transmission assembly both include a same rack.

In some embodiments, the puncture core assembly also includes a suture needle fixing mechanism and a suture thread release mechanism, the suture needle fixing mechanism includes a suture needle fixing operating assembly, the suture thread release mechanism includes a suture thread release operating assembly, and the suture needle fixing operating assembly, the suture operating assembly and the suture thread release operating assembly are the same operating assembly.

In some embodiments, the same operating assembly includes a pressing cover, which is pressed or pulled to move the same operating assembly downwardly or upwardly.

Some embodiments of the present invention provide a misoperation prevention mechanism, wherein the first part includes a wall, and the second part includes a blocking part. The misoperation prevention mechanism includes the wall and the blocking part. In an initial position, the blocking part is butted against the wall to prevent the first part from moving relative to the second part due to misoperation. The wall includes a notch. When the first part is rotated and located in another position, the notch contains the blocking part, and then the first part is operated to move relative to the second part.

Some embodiments of the present invention provide a misoperation prevention mechanism, the first part includes an adjusting block and a spring, one end of the spring is butted against the adjusting block, the other end of the spring butts against other parts of the first part, except the adjusting block, and the elastic force of the spring prevents the adjusting block from moving relative to the other parts. The second part includes a blocking part. The misoperation prevention mechanism includes the adjusting block, the spring and the blocking part. The blocking part has a first guide inclined plane, and the adjusting block has a second guide inclined plane. In an initial position, the first guide inclined plane is butted against the second guide inclined plane. When a force of misoperation of the first part is not enough to overcome the elastic force of the spring, the blocking part is butted against the adjusting block to prevent the first part from moving relative to the second part due to misoperation. When an operating force is increased, the force overcomes the elastic force of the spring to move the adjusting block towards the other part of the second part along the guide inclined plane. When the adjusting block is separated from the blocking part, the first part is operated to move relative to the second part.

In some embodiments, the first part also includes another adjusting block, and the other end of the spring is butted against the another adjusting block.

In some embodiments, the first part includes a guide groove, the adjusting block includes a protruding part, and the protruding part is movably contained in the guide groove.

Some embodiments of the present invention provide a puncture core assembly, wherein the puncture core assembly includes a suture mechanism. The suture mechanism is the above suture mechanism for the puncture core assembly.

In some embodiments, the puncture core assembly also includes a positioning mechanism. The positioning mechanism is the positioning mechanism for the above puncture core assembly.

In some embodiments, the puncture core assembly also includes a suture thread release mechanism. The suture thread release mechanism is the suture thread release mechanism for the puncture core assembly according to any one of the above.

In some embodiments, the puncture core assembly also includes a suture needle fixing mechanism. The suture needle fixing mechanism is the above suture needle fixing mechanism for the puncture core assembly.

In some embodiments, the puncture core assembly also includes a misoperation prevention mechanism. The misoperation prevention mechanism is the above misoperation prevention mechanism for the puncture core assembly.

Some embodiments of the present invention provide a suturable puncture device, wherein the suturable puncture device includes a puncture core assembly and a cannula assembly. The puncture core assembly is detachably sleeved on the cannula assembly. The puncture core assembly is the above puncture core assembly.

Some embodiments of the present invention provide a use method of a suturable puncture device. The suturable puncture device includes a puncture core assembly and a cannula assembly. The puncture core assembly is detachably sleeved on the cannula assembly. The puncture core assembly includes a first operating assembly, a second operating assembly, a third operating assembly, a positioning blade, a rod wall pipe, a suture member, a suture thread release mechanism and a suture thread, and the suture member includes a suture needle. The use method includes the following steps.
(1) The first operating assembly is operated, so that the positioning blade rotates outwardly, and the positioning blade protrudes out of the outer surface of the rod wall pipe.
(2) The puncture core assembly or the suturable puncture device is pulled upwardly, and the positioning blade realizes positioning.
(3) The second operating assembly is operated, so that the suture member rotates.
(4) The third operating assembly is operated, so that the suture thread release mechanism is opened, and the suture thread contained in the suture thread release mechanism is released.
(5) The suture member rotates in place, and the suture needle is fixed.
(6) The suture needle is further fixed.
(7) The second operating assembly is operated, so that the suture member returns.
(8) The third operating assembly is operated, so that the suture thread release mechanism is closed.
(9) The first operating assembly is operated, so that the positioning blade rotates inwardly.
(10) The puncture core assembly or the suturable puncture device is pulled upwardly.

In some embodiments, in the step (1), the operation is rotation.

In some embodiments, the first operating assembly includes a turntable. The step (1) also includes: the turntable is rotated. The turntable rotates along the first circumferential direction to drive a first transmission assembly to rotate, and the first transmission assembly drives the positioning blade to rotate outwardly.

In some embodiments, the step (1) also includes: a suture channel is exposed after the positioning blade rotates outwardly.

In some embodiments, in the step (3) and the step (4), all the operations are pressing operations.

In some embodiments, in the step (3) and the step (4), the second operating assembly and the third operating assembly are the same operating assembly.

In some embodiments, the puncture core assembly includes a suture assembly. The suture assembly includes the suture member. The suture member also includes a gear. The second operating assembly includes a pressing cover. The step (3) also includes: the pressing cover is pressed to enable the pressing cover to move downwardly. The pressing cover moves downwardly to drive a second transmission assembly to move downwardly, and a rack of the second transmission assembly moves downwardly to drive the gear to rotate so as to enable the suture member to rotate.

In some embodiments, the third operating assembly includes a pressing cover. The step (4) also includes: the pressing cover is pressed to enable the pressing cover to move downwardly. The pressing cover moves downwardly to drive a transmission assembly to move downwardly, and a rack of the transmission assembly moves downwardly to drive a toothed member of the suture thread release mechanism to rotate so as to open the suture thread release mechanism.

In some embodiments, the third operating assembly includes a pressing cover. The step (4) also includes: the pressing cover is pressed to enable the pressing cover to move downwardly. The pressing cover moves downwardly to drive a transmission assembly to move downwardly, and a rack of the transmission assembly moves downwardly to drive a toothed member of the suture thread release mechanism to rotate so as to open the suture thread release mechanism. The second transmission assembly in the step (3) and the transmission assembly in the step (4) are the same transmission assembly, the rack in the step (3) and the rack in the step (4) are the same rack, and a rotation of the suture needle is synchronized with the opening of the suture thread release mechanism.

In some embodiments, the step (5) also includes: the suture needle is held by a receiving member to be fixed.

In some embodiments, the step (6) also includes: the second operating assembly is pressed. The second operating assembly includes a pressing cover, the pressing cover downwardly pushes a third transmission assembly, the third transmission assembly pushes a movable member to move downwardly, and a slit of the movable member holds the suture needle.

In some embodiments, in the step (7) and the step (8), all the operations are pulling.

In some embodiments, in the step (7) and the step (8), the second operating assembly and the third operating assembly are a same operating assembly.

In some embodiments, the second operating assembly includes a pressing cover. The step (7) also includes: the pressing cover is pulled to enable the pressing cover to move upwardly. The pressing cover moves upwardly to drive a second transmission assembly to move upwardly, and a rack of the second transmission assembly moves upwardly to drive a gear of the suture assembly to rotate so as to rotate and return the suture member.

In some embodiments, the third operating assembly includes a pressing cover. The step (8) also includes: the pressing cover is pulled to enable the pressing cover to move upwardly. The pressing cover moves upwardly to drive a transmission assembly to move upwardly, and a rack of the transmission assembly moves upwardly to drive a toothed member of the suture thread release mechanism to rotate so as to close the suture thread release mechanism.

In some embodiments, in the step (9), The first operating assembly being operated is The first operating assembly is rotated.

In some embodiments, the first operating assembly includes a turntable. The step (9) also includes: the turntable is rotated. The turntable rotates along the second circumferential direction to drive a first transmission assembly to rotate, and the first transmission assembly drives the positioning blade to rotate inwardly.

In some embodiments, in the step (3) and the step (4), the second operating assembly and the third operating assembly are the same operating assembly. The third operating assembly includes a pressing cover. The step (4) also includes: the pressing cover is pressed to enable the pressing cover to move downwardly. The pressing cover moves downwardly to drive a transmission assembly to move downwardly, and a rack of the transmission assembly moves downwardly to drive a toothed member of the suture thread release mechanism to rotate so as to open the suture thread release mechanism. The second transmission assembly in the step (3) and the transmission assembly in the step (4) are the same transmission assembly, the rack in the step (3) and the rack in the step (4) are the same rack, and the step (3) and the step (4) are executed synchronously.

### Brief Description of the Drawings

Fig. 1 illustrates a schematic structural diagram of a puncture device provided by the present invention.
Fig. 2 illustrates a schematic structural diagram of a puncture core assembly provided by the present invention.
Fig. 3 illustrates an explosive view of Fig. 2.
Fig. 4 illustrates a schematic structural diagram of a part required to achieve positioning when positioning is not performed.
Fig. 5 illustrates a connection schematic diagram of a rotating ring and a positioning member.
Fig. 6 illustrates a schematic structural diagram of a part required to achieve positioning after positioning is performed.
Fig. 7 illustrates a schematic structural diagram of a part driven by a pressing cover to move.
Fig. 8 illustrates a schematic structural diagram of a pressing cover in an angle.
Fig. 9 illustrates a schematic structural diagram of Fig. 8 in another angle.
Fig. 10 illustrates a schematic structural diagram of an adjusting frame and an adjusting block.
Fig. 11 illustrates a schematic diagram of a suture assembly when withdrawing of a needle is not performed.
Fig. 12 illustrates a schematic structural diagram of a receiving member.
Fig. 13 illustrates a schematic structural diagram of a puncture core assembly when a needle withdrawing action, a needle receiving action and a suture thread releasing action are completed.
Fig. 14 illustrates a sectional view of a puncture core assembly in the state of Fig. 12.
Fig. 15 illustrates a schematic structural diagram of a part required to achieve a needle receiving action and a needle fixing action.
Fig. 16 illustrates a schematic structural diagram of a driving pressing disc in a second position.
Fig. 17 illustrates a schematic structural diagram of a needle fixing assembly when a needle fixing action is completed.
Fig. 18 illustrates a schematic structural diagram of a puncture core assembly after a suture assembly is reset.

Reference numerals:
1-operating assembly,
11-turntable, 111-lower housing, 112-toggle arm, 113-blocking sheet, 113a-first guide inclined plane,
12-pressing cover, 121-pressing disc, 122-notch, 122a-slit, 1221-rib plate group, 123-circumferential wall, 124-push member, 1241-push member plate,
2-butting disc, 21-central through hole,
3-rod wall pipe, 31-window, 32-deformation sheet,
4-transmission assembly,
41-first transmission assembly, 411-first transmission pipe, 412-transmission arm, 413-rotating ring,
42-second transmission assembly, 421-adjusting frame, 4212-guide groove, 422-adjusting block, 422a-second guide inclined plane, 4221-guide protruding block, 4222-anti-shaking protruding strip, 423-elastic member, 424-transmission rod, 424a-near end, 424b-far end, 425-rack, 425a-upper toothed segment, 425b-lower toothed segment,
43-third transmission assembly, 431-upper transmission ring, 432-connecting rod, 433-third transmission pipe, 434-lower transmission ring, 435-booster arm,
5-supporting assembly, 5'-upper half part, 5"-lower half part, 51-first supporting member, 511-avoidance space, 52-second supporting member, 53-suture channel, 54-avoidance groove, 541,542-groove wall, 55-containing hole,
6-execution assembly,
61-positioning assembly, 611-movement aiding arm, 6111-waist-shaped groove, 612-pivot shaft, 613-positioning blade, 614-protruding part,
62-suture assembly, 621,622-suture member, 623-gear, 624-rotating shaft, 625-first suture arm, 626-second suture arm, 627-suture needle,
63-receiving assembly, 631,632-receiving member, 6311-receiving part, 6312-holding part, 6313-receiving sheet, 6314-blocking arm,
64-needle fixing assembly, 641,642-movable member, 641a-limiting slit,
7-puncture tip, 71-first wall housing, 72-second wall housing, 711,721-toothed member, 7111-first limiting protruding block, 712-first supporting shaft, 722-second supporting shaft, 73-protrusion,
8-insertion block assembly, 81-button, 82-clamping hook, 83-spring,
9-cover body, 91-cylinder, and 92-circumferential through hole.

### Detailed Description of the Embodiments

The embodiments of the present invention will be described below in detail and examples of the embodiments are shown in the drawings. In the description of the present invention, "several" means at least one, unless otherwise expressly and specifically defined.

The embodiments described below with reference to the drawings are exemplary and intended to explain the present invention and should not be understood as limits to the present invention.

In order to simplify the description, in the embodiment of the present invention, the ends of all part close to a doctor are set as "near ends" or "above", and the ends away from the doctor, that is, the ends close to the body of a patient are set as "far ends" or "below". Connection includes both direct connection and indirect connection. Connection includes fixed connection, movable connection, separable connection and the like, unless otherwise definitely limited.

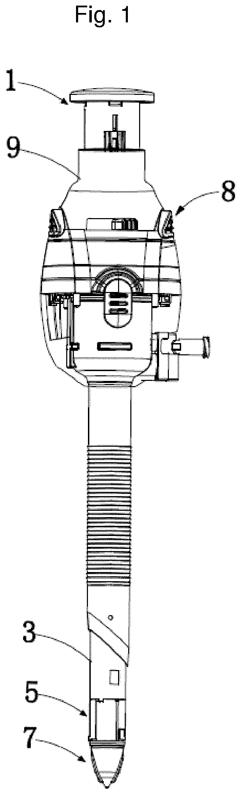

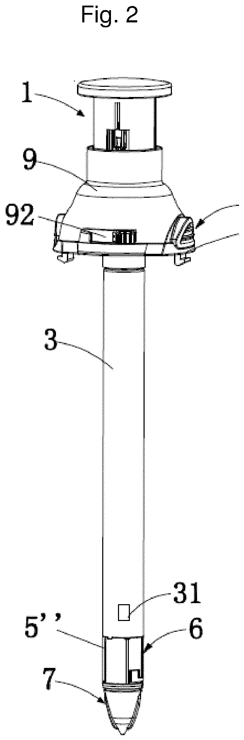

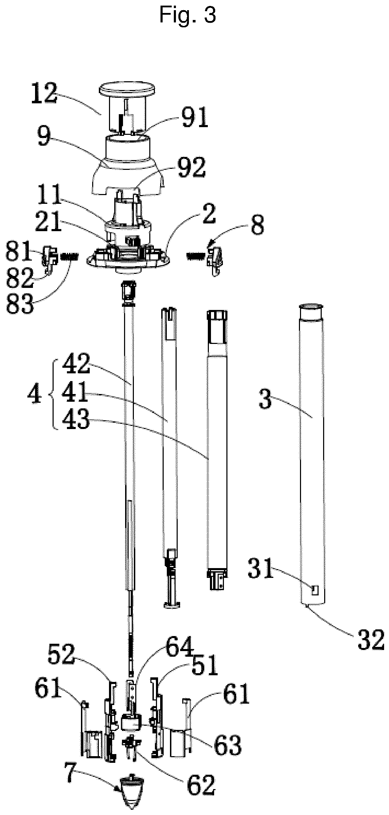

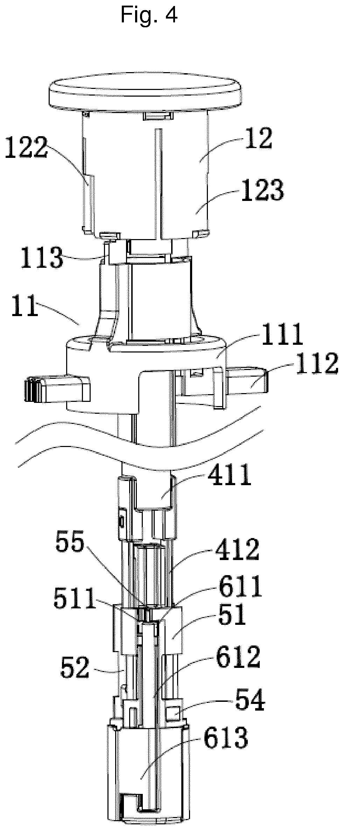

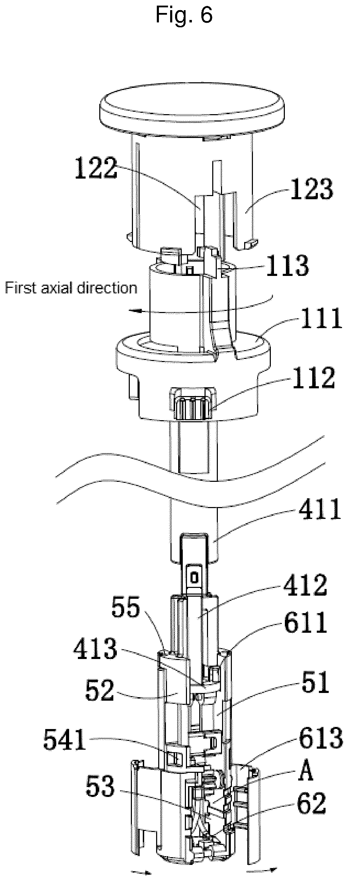

Referring to Fig. 1, some embodiments of the present invention provide a puncture device, which includes a cannula assembly (not labeled) and a puncture core assembly partly disposed in the cannula assembly in a sleeving manner. Referring to Fig. 2 and Fig. 3, the puncture core assembly includes an operating assembly 1, a butting disc 2, a rod wall pipe 3, a transmission assembly 4, a supporting assembly 5, an execution assembly 6, a puncture tip 7, an insertion block assembly 8 and a cover body 9. The cover body 9 covers the upper surface of the butting disc 2 to prevent foreign matters from falling into the butting disc 2 and affecting the realization of the function of the puncture core assembly. A cylinder 91 is disposed in the center of the cover body 9, and the cylinder 91 is sleeved on the outside of the operating assembly 1. The part of the operating assembly 1 exposed to the cylinder 91 is used for being manipulated by a doctor. Inside the cylinder 91, the operating assembly 1 is connected with the transmission assembly 4. The center of the butting disc 2 is provided with a central through hole 21 coaxial with the cylinder 91, and the transmission assembly 4 is disposed in the central through hole 21 in a penetrating manner. The supporting assembly 5 is below the transmission assembly 4, and the supporting assembly 5 is used to support and protect the execution assembly 6. The supporting assembly 5 includes two supporting members with the same structure, namely the first supporting member 51 and the second supporting member 52. The rod wall pipe 3 is sleeved on the outside of the transmission assembly 4, the near end of the rod wall pipe 3 is connected to the lower surface of the butting disc 2, and the rod wall pipe 3 is coaxial with the central through hole 21. The far end of the rod wall pipe 3 surrounds an upper half part 5' of the supporting assembly 5 and is fixedly connected with the upper half part 5'. A lower half part 5" of the supporting assembly 5 is exposed between the rod wall pipe 3 and the puncture tip 7, and the outer surface of the lower half part 5" is flush with the outer surface of the rod wall pipe 3, so that the puncture core assembly enters and exits a puncture opening without hindrance. The outer surface of the rod wall pipe 3 is the outer surface of the puncture core assembly. The end face of the far end of the rod wall pipe 3 extends downwardly to form two symmetrical deformation sheets 32. The junction of the upper half part 5' and the lower half part 5" is provided with an avoidance groove 54. As illustrated in Fig. 4 and Fig. 6, a groove wall 541 of the avoidance groove 54 is located between the end face of the far end of the deformation sheet 32 and the end face of the far end of the rod wall pipe 3. When the rod wall pipe 3 is sleeved on the upper half part 5', the avoidance groove 54 provides a space for the deformation sheet 32 to bend. A deformation tool is used to bend the deformation sheet 32 inwardly to form a clamping hook (not shown in the figures) and make the clamping hook been abutted aginst the groove wall 541. In this way, the groove wall 541 prevents the clamping hook from moving axially, thereby preventing the rod wall pipe 3 from moving axially. Moreover, the rod wall pipe 3 is provided with at least one first pin hole (not shown in the figures), and the upper half part of the supporting assembly 5 is provided with at least one second pin hole (not shown in the figures). When the rod wall pipe 3 is sleeved on the outside of the upper half part of the supporting assembly 5, the first pin hole and the second pin hole are made to be coaxial, a fixed pin (not shown in the figures) is used to be sequentially inserted into the first pin hole and the second pin hole which are coaxial, thus, the rod wall pipe 3 is fixed with the upper half part of the supporting assembly 5, and then the rod wall pipe 3 is connected with the supporting assembly 5. The cannula assembly is sleeved on the outside of the rod wall pipe 3, and the near end of the cannula assembly is separably connected with the lower surface of the butting disc 2. The insertion block assembly 8 is used to realize the separable connection. The insertion block assembly 8 includes two symmetrical insertion block members (not labeled), and each insertion block member includes a button 81, a clamping hook 82 and a spring 83. The button 81 is movably connected to the upper part of the butting disc 2, the clamping hook 82 is fixedly connected to the button 81 and extends from the button 81 to the lower part of the butting disc 2, the spring 83 is located in the cover body 9, one end of the spring 83 is abutted against the button 81, and the other end of the spring 83 is abutted against the vertical part of the butting disc 2. The clamping hook 82 is clamped with the cannula assembly. The button 81 and the spring 83 are used to move the clamping hook 82 to separate the clamping hook 82 from the cannula assembly, so as to separate the butting disc 2 from the near end of the cannula assembly. The insertion block assembly 8 is the traditional art and will not be elaborated. In the present invention, based on the positional relationship shown in Fig. 2, the end where the puncture tip 7 is located in the puncture core assembly is called the far end or below, the end where the operating assembly 1 is located in the puncture core assembly is called the near end or above, the side close to the central axis of the rod wall pipe 3 is called the inner side, the side away from the central axis of the rod wall pipe 3 is called the outer side, the radius direction of the rod wall pipe 3 is called the radial direction, the direction of the central axis of the rod wall pipe 3 is called the axial direction, the direction perpendicular to the axial direction is called the transverse direction, the transverse direction includes the radial direction, the direction perpendicular to the central axis of the rod wall pipe 3 and parallel to the circumference of the rod wall pipe 3 is defined as the circumferential direction, and the surface parallel to the circumferential direction is defined as the circumferential surface. The central axis of the rod wall pipe 3 is the central axis of the puncture core assembly. The above directions of the rod wall pipe 3 are the corresponding directions of the puncture core assembly, and the above surfaces of the rod wall pipe 3 are the corresponding surfaces of the puncture core assembly. The outer surface of the puncture core assembly refers to the outer surface of the rod wall pipe 3.

During an operation, a doctor cuts a small incision in the abdomen of a patient at first, uses the puncture tip 7 of the puncture core assembly provided by the present invention to puncture human tissue to form a puncture opening, inserts the lower part of the cannula assembly into the human body, separates the puncture core assembly from a cannula by pressing the insertion block assembly 8, pulls out the puncture core assembly from the cannula, and then extends a surgical instrument into the cannula to perform an operation. After the operation is completed, the doctor takes out the surgical instrument, inserts the puncture core assembly into the cannula again, clamps the puncture core assembly with the cannula assembly through the insertion block assembly 8 again, and starts a suture operation. According to the operation sequence, the suture operation process using the puncture core assembly provided by the present invention includes the following actions or steps: positioning, forming a suture channel, suturing out a needle, releasing a suture thread, receiving a needle, fixing the needle, and resetting. Further, after the puncture core assembly is reset, the doctor pull out the puncture core assembly and tie the suture thread. The operation of suturing out the needle and the operation of releasing the suture thread are started synchronously, and the operation of releasing the suture thread is completed before the operation of suturing out the needle.

Referring to Fig. 3, the doctor manipulates the operating assembly 1 to generate a driving force, and the driving force is transmitted to the execution assembly 6 and the puncture tip 7 by the transmission assembly 4 to enable the execution assembly 6 and the puncture tip 7 to perform a suture operation. The operating assembly 1 includes a pressing cover 12 and a turntable 11 from top to bottom, the transmission assembly 4 includes a second transmission assembly 42, a first transmission assembly 41 and a third transmission assembly 43 disposed from inside to outside in a sleeving manner, and the execution assembly 6 includes a positioning assembly 61, a suture assembly 62, a receiving assembly 63 and a needle fixing assembly 64. Specifically, the turntable 11 is manipulated to rotate, the turntable 11 rotates to drive the first transmission assembly 41 to rotate, the first transmission assembly 41 drives the positioning assembly 61 to rotate, and the positioning assembly 61 rotates to perform a positioning action and form the suture channel, so that the positioning action is synchronized with the formation of the suture channel. The pressing cover 12 is manipulated to move, the pressing cover 12 moves to drive the second transmission assembly 42 and the third transmission assembly 43 to move, the second transmission assembly 42 moves to synchronously drive the suture assembly 62 to act and drives the puncture tip 7 to open, the suture assembly 62 acts to perform the action of suturing out the needle, and the puncture tip 7 opens to perform a suture thread releasing action, so that the needle withdrawing action and the suture thread releasing action are synchronized. The needle receiving assembly 63 does not need to be driven, and the needle receiving assembly 63 performs a needle receiving action. The third transmission assembly 43 moves to drive the needle fixing assembly 64 to move, and the needle fixing assembly 64 moves to perform a needle fixing action. Reset refers to the reset of the positioning assembly 61, the suture assembly 62 and the puncture tip 7. A positioning operating assembly includes a turntable 11, a suture operating assembly, a release suture thread operating assembly or a suture needle fixing operating assembly all include a pressing cover 12, and the positioning assembly includes a positioning execution assembly. The suture assembly includes a suture execution assembly, and the suture execution assembly includes a suture needle. The puncture tip is called a release suture thread execution assembly. The first transmission assembly is called a positioning transmission assembly, the second transmission assembly is called a suture transmission assembly and a release suture thread transmission assembly, and the third transmission assembly is called a suture needle fixing transmission assembly.

Referring to Fig. 4 and Fig. 6, the turntable 11 is an integrally formed part including a housing 111 and a toggle arm 112. The housing 111 is covered above the butting disc 2 to protect the transmission assembly 4 above the central through hole 21. The cover body 9 covers the housing 111. The outer end of the toggle arm 112 is located outside the cover body 9, the toggle arm 112 sequentially passes through a circumferential through hole 92 of the cover body 9 and the housing 111, and the inner end of the toggle arm 112 is located inside the housing 111. Referring to Fig. 3, the circumferential through hole 92 has a space for the toggle arm 112 to move circumferentially. Also referring to Fig. 4, the outer end of the toggle arm 112 is an operating end, and the inner end is clamped with a first transmission pipe 411 of the first transmission assembly 41. The first transmission assembly 41 is sleeved in the rod wall pipe 3. The first transmission assembly 41 sequentially includes the first transmission pipe 411, two symmetrical transmission arms 412 and a rotating ring 413 from top to bottom. The first transmission pipe 411 is a hollow pipe extending along the axial direction. The near end of the first transmission pipe 411 is located above the central through hole 21 and is clamped with the inner end of the toggle arm 112. The far end of the first transmission pipe 411 is fixedly connected with the near end of the transmission arm 412. The two symmetrical transmission arms 412 extend axially, the near end of the transmission arm 412 is fixed on the outside of the first transmission pipe 411, and the far end of the transmission arm 412 is integrally formed with the rotating ring 413. The rotating ring 413 is located between the first supporting member 51 and the second supporting member 52, and the rotating ring 413 is drivably connected with the positioning assembly 61.

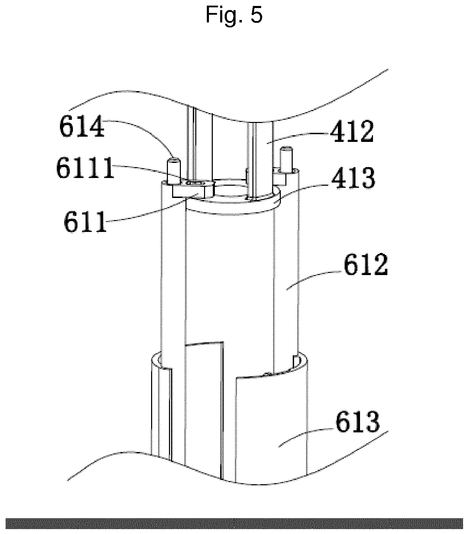

The positioning assembly 61 includes two symmetrically disposed positioning members (not labeled) with the same structure, one positioning member is pivotally connected with the first supporting member 51 and the other positioning member is pivotally connected with the second supporting member 52. An upper end of each positioning member has a protruding part 614 extending upwardly along the axial direction, the lower end has a protruding part 614 extending downwardly along the axial direction, both the first supporting member 51 and the second supporting member 52 have containing holes 55 extending upwardly and downwardly along the axial direction, the protruding part 614 is contained in the corresponding containing hole 55 and rotates in the containing hole 55, thus, one positioning member is pivotally connected with the first supporting member 51, and the other positioning member is pivotally connected with the second supporting member 52. For simplicity of description, only the positioning member pivotally connected to the first supporting member 51 is described below. The positioning member includes a movement aiding arm 611, a pivot shaft 612 and a positioning blade 613. The pivot shaft 612 extends axially, the movement aiding arm 611 is perpendicular to the pivot shaft 612, and the movement aiding arm 611 is drivably connected with the rotating ring 413, so as to realize the rotation of the rotating ring 413 to drive the movement aiding arm 611 to rotate with a connecting line between the upper and lower protruding parts 614 as an axis (i.e. the axis where the pivot shaft 612 is located), and further realize the rotation of the rotating ring 413 to drive the positioning member to rotate with the pivot shaft 612 as the axis. The drivable connection means that the rotation of the rotating ring 413 drives the movement aiding arm 611 to rotate. Specifically, the rotating ring 413 is provided with an upward protruding part (also known as a movement aiding body) (not shown in the figures). As illustrated in Fig. 5, the movement aiding arm 611 has a waist-shaped groove 6111, the movement aiding body is contained in the waist-shaped groove 6111, and the movement aiding body moves in the waist-shaped groove 6111. When the rotating ring 413 rotates in the circumferential direction, the movement aiding body also rotates in the circumferential direction. The movement aiding body rotates in the circumferential direction to move it in the waist-shaped groove 6111 and drive the waist-shaped groove 6111 to rotate with the pivot shaft 612 as the axis. The movement aiding arm 611 where the waist-shaped groove 6111 is located also rotates with the pivot shaft 612 as the axis, so that the positioning blade 613 rotates outwardly and inwardly with the pivot shaft 612 as the axis. The first supporting member 51 includes an avoidance space 511, the movement aiding arm 611 is located in the avoidance space 511, and one end of the movement aiding arm 611 is integrally formed with the near end of the pivot shaft 612. The pivot shaft 612 extends axially, and the positioning blade 613 is integrally formed at the far end of the pivot shaft 612. When the positioning blade 613 does not rotate (i.e., in the closed state), the positioning blade 613 is flush with the outer surface of the rod wall pipe 3 and the outer surface of the first supporting member 51. When the positioning blade 613 rotates (i.e., in the open state), the positioning blade 613 is protruded out of the outer surface of the rod wall pipe 3 and the outer surface of the first supporting member 51. Therefore, after rotation, the two positioning blades 613 protrude out of the outer surface of the rod wall pipe 3 and are abutted against tissue on both sides of the puncture opening, so as to realize a positioning function. At this time, the suture assembly 62 is in a suturable position. Since the upper surface of the positioning blade 613 and the tissue are in surface contact, and the upper surface of the positioning blade 613 is perpendicular to the axis of the puncture core assembly, the positioning is more accurate. Since the positioning blade 613 rotates with one axial direction of the puncture core assembly as the central axis, the contact area between the positioning blade 613 and the tissue is not affected by the position of the positioning blade 613 after rotation. Since the upper surface of the positioning blade 613 and the tissue are in surface contact, instead of point contact, the positioning is more accurate.

When the turntable 11 does not rotate but in the initial position, the toggle arm 112 is located at one end of the circumferential through hole 92. The doctor toggles the toggle arm 112 along the first circumferential direction, the toggle arm 112 rotates in the circumferential through hole 92 along the first circumferential direction, the toggle arm 112 drives the first transmission assembly 41 to rotate along the first circumferential direction, the first transmission pipe 411 rotates along the first circumferential direction, the first transmission pipe 411 drives the transmission arm 412 to move along the first circumferential direction, the transmission arm 412 drives the rotating ring 413 to rotate along the first circumferential direction, the rotating ring 413 drives the two symmetrical movement aiding arms 611 of the positioning assembly 61 to rotate, the two symmetrical movement aiding arms 611 respectively drive the two pivot shafts 612 to rotate, the two pivot shafts 612 respectively drive the two positioning blades 613 to pivot outwardly, so that the two positioning blades 613 change from a state flush with the outer surface of the rod wall pipe 3 to a state protruding out of the outer surface of the rod wall pipe 3, that is, from the closed state to the open state, and the two positioning blades 613 are protruded out of the outer surface of the rod wall pipe 3 and are abutted against the tissue on both sides of the puncture opening, so as to realize the positioning function. When the shift arm 112 rotates to the other end of the circumferential through hole 92, the hole wall of the circumferential through hole 92 prevents the toggle arm 112 from continuing to rotate along the first circumferential direction, so that the positioning blade 613 stops rotating, and at this time, the turntable 11 is in the termination position. When the positioning assembly 61 performs a reset action, the toggle arm 112 is shifted along a second circumferential direction, and the first circumferential direction is opposite to the second circumferential direction. According to the above action transmission relationship, the first transmission assembly 41 rotates along the second circumferential direction to drive the two pivot shafts 612 to rotate reversely, and the two pivot shafts 612 respectively drive the two positioning blades 613 to pivot inwardly, thus, the two positioning blades 613 are restored from a state protruding out of the outer surface of the rod wall pipe 3 to a state flush with the outer surface of the rod wall pipe 3, that is, from the open state to the closed state, so as to realize the reset of the positioning assembly 61.

There is a space between the first supporting member 51 and the second supporting member 52, which is defined as a containing space A for containing the suture assembly 62. When the positioning blade does not rotate, the positioning blade closes the part where the suture assembly 62 is located in the containing space A. After the positioning blade rotates, the part where the suture assembly 62 is located in the containing space A is exposed to expose a suture channel 53. The suture assembly 62 moves to the outside of the rod wall pipe 3 through the suture channel 53 to perform a needle withdrawing action.

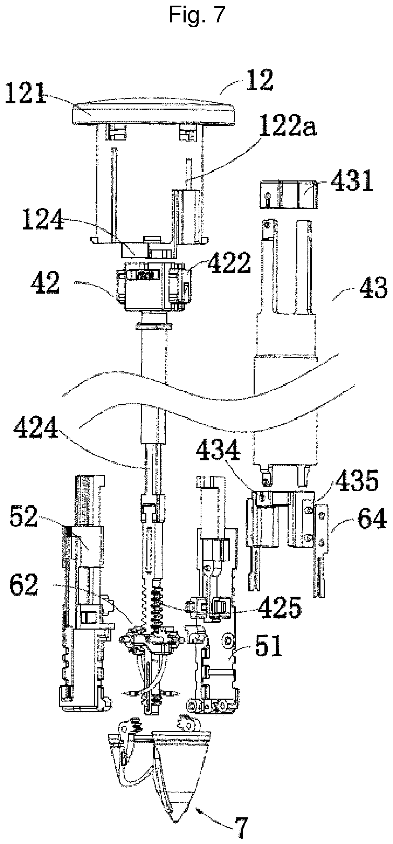

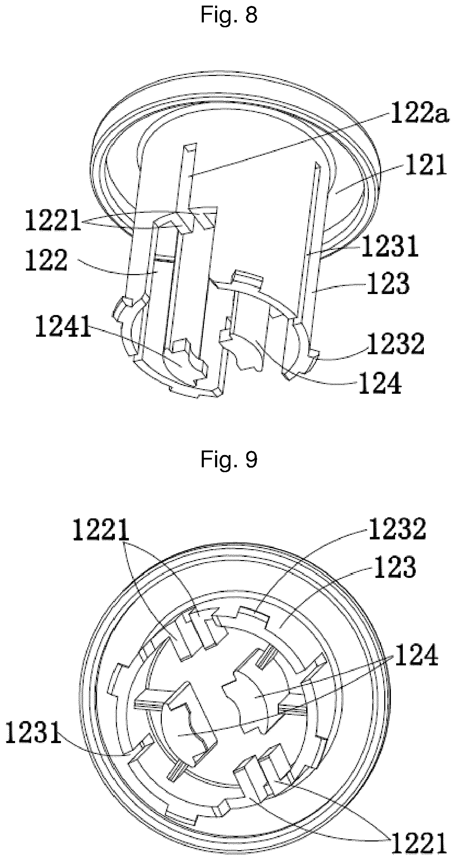

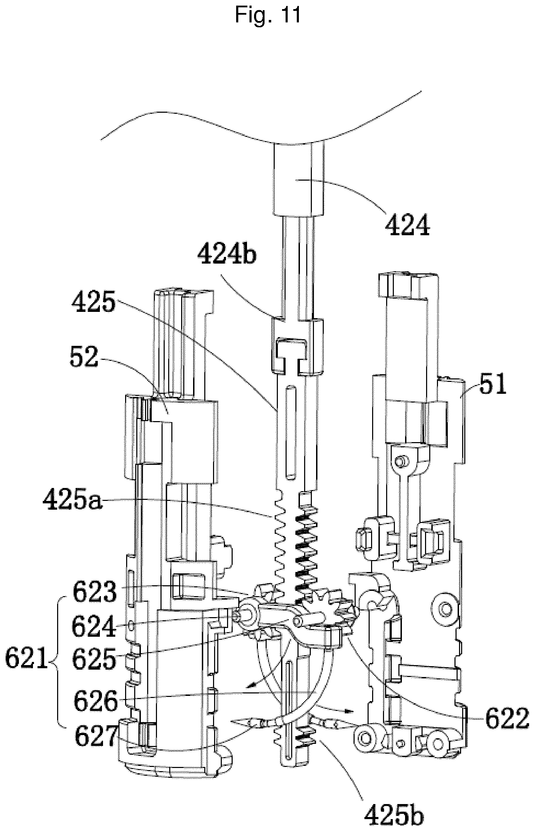

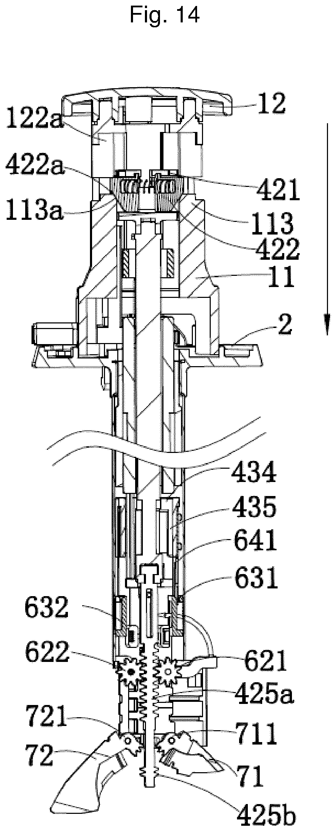

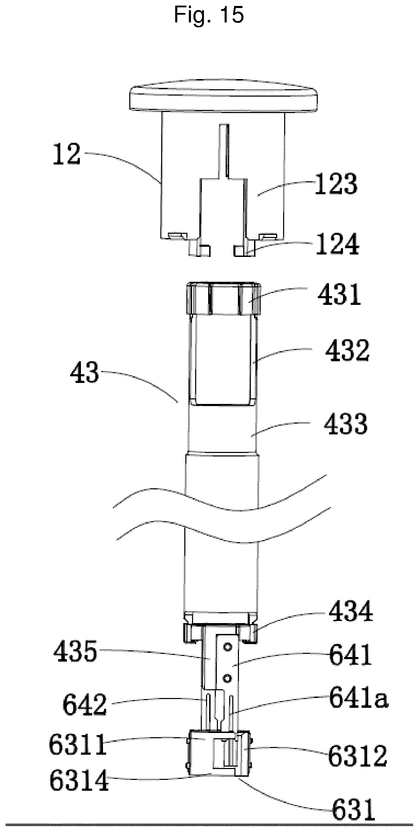

Referring to Figs. 7-9, the pressing cover 12 includes a pressing disc 121, a circumferential wall 123 and two symmetrical push feet 124, the pressing disc 121 is exposed to the cover body 9, and the near end of the circumferential wall 123 and the near ends of the two push feet 124 are integrally formed on the lower surface of the pressing disc 121 and extend axially. The circumferential wall has two symmetrical notches 122, both of which are formed after removing a part of the circumferential wall 123. The notch 122 includes two slits 122a extending along the axial direction. Both sides of the inner side of each slit 122a are protruded inwardly along the radial direction to form two rib plates. The above two rib plates are called a group of rib plates. Each group of rib plates is defined as a rib plate group 1221, and the two rib plate groups 1221 and the two push feet 124 are disposed in a staggered manner. An axial length of each push member 124 is greater than an axial length of the circumferential wall 123, and the far end of the push member 124 protrudes inwardly along the radial direction to form a push member plate 1241. When the pressing cover 12 is not pressed and is in the first position, each rib plate group 1221 is abutted against an adjusting block 422 of the second transmission assembly 42. When the pressing cover 12 is pressed, the rib plate group 1221 presses down the adjusting block 422, so that the second transmission assembly 42 where the adjusting block 422 is located moves downwardly. As illustrated in Figs. 11-13, the second transmission assembly 42 moves downwardly to drive the suture assembly 62 to perform the needle withdrawing action, and the puncture tip 7 to perform the suture thread releasing action. When the needle withdrawing action of suturing is completed, as illustrated in Fig. 14, the pressing cover 12 moves to a second position. The pressing cover 12 is continuously pressed. After the pressing cover 12 moves for an idle stroke, the two push member plates 1241 are abutted against an upper transmission ring 431 of the third transmission assembly 43. The pressing cover 12 is continuously pressed, so that the push member plate 1241 presses down the upper transmission ring 431, so that the third transmission assembly 43 where the upper transmission ring 431 is located moves downwardly. As illustrated in Figs. 15-17, the third transmission assembly 43 moves downwardly to drive the needle fixing assembly 64 to perform the needle fixing action. After the needle fixing action is completed, the pressing cover 12 moves to a third position. The suture assembly, the puncture tip and the needle fixing assembly share the pressing cover 12 for operation, which reduces the number of operating assemblies and improves the operation convenience.

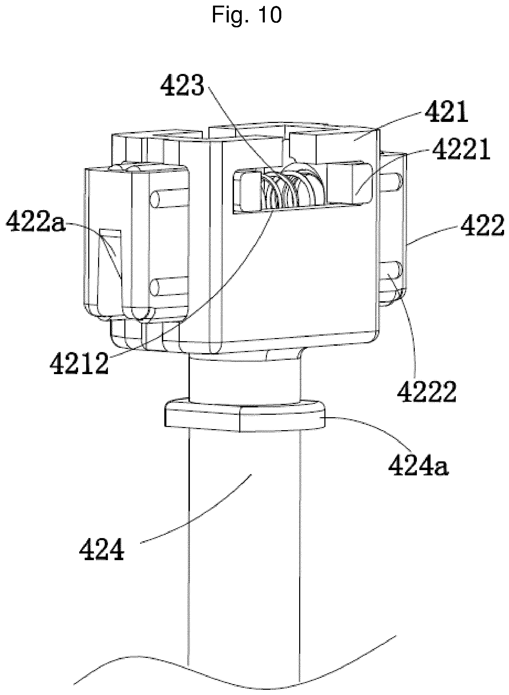

Referring to Fig. 7, the second transmission assembly 42 includes an adjusting part (not labeled), a transmission rod 424, and a rack 425. Referring to Fig. 10, the adjusting part includes an elastic member 423, an adjusting frame 421 and two symmetrical adjusting blocks 422. The elastic member 423 is a spring, the elastic member 423 is located between the two adjusting blocks 422, each adjusting block 422 is disposed, so that a part of the adjusting block 422 moves radially between the inside and outside of the adjusting frame 421, and the middle part of the adjusting frame 421 is contained between the two push feet 124. When the pressing cover 12 is in the first position and the second position, and in the process of moving from the first position to the second position, due to the elastic force of the elastic member 423, a certain distance is maintained between the two adjusting blocks 422, a part of the adjusting block 422 is exposed to the adjusting frame 421, the adjusting part is in the first state, and each rib plate group 1221 is abutted against the adjusting block 422 on the same side. When the pressing cover 12 moves downwardly from the second position to the third position, the elastic member 423 shrinks, the adjusting block 422 is contained in the adjusting frame 421, and the adjusting part is in the second state. The downward extending cylindrical part of the adjusting frame 421 is fixedly connected with the near end 424a of the transmission rod 424. The near end 424a of the transmission rod 424 is located above the two push member plates 1241, and the radial distance between the two push member plates 1241 is less than the width of the near end 424a. In this way, when the pressing cover 12 is pulled, the push member plate 1241 is abutted against the near end 424a of the transmission rod 424, thus, the second transmission assembly 42 where the near end 424a is located is pulled to move upwardly, and the second transmission assembly 42 moves upwardly to drive the suture assembly 62 and the puncture tip 7 to perform the reset action. The transmission rod 424 extends downwardly in the axial direction and passes through the first transmission assembly 41 to form a far end 424b, which is connected to the rack 425. Teeth are disposed on both sides of the rack 425. The teeth on both sides are divided into two segments from top to bottom, namely, an upper tooth segment 425a and a lower tooth segment 425b. Teeth are symmetrically disposed on both sides of the upper tooth segment 425a. The upper tooth segment 425a transmits a driving force to the suture assembly 62 to drive the suture assembly 62 to perform the needle withdrawing action of suturing. Teeth are disposed on at least one side of the lower tooth segment 425b, and the lower tooth segment 425b transmits the driving force to the puncture tip 7 to drive the puncture tip 7 to perform the suture thread releasing action.

Referring to Fig. 11, the suture assembly 62 includes suture members 621 and 622 with the same structure. For simplicity of description, only the suture member 621 is described below. The suture member 621 includes a gear 623, a rotating shaft 624, a first suture arm 625, a second suture arm 626, and a suture needle 627. The teeth on one side of the upper tooth segment 425a are engaged with the gear 623. The gear 623 is disposed on the rotating shaft 624 in a sleeving manner, and the gear 623 is fixed on the rotating shaft 624. One end of the rotating shaft 624 is rotatably connected with the first supporting member 51, and the other end is rotatably connected with the second supporting member 52. The rotating shaft 624 is fixed with one end of the first suture arm 625. The first suture arm 625 is an arm with an avoidance bending part, the avoidance bending part is used to give way to the rotating shaft of the suture member 622, so that the rotating shaft of the suture member 622 and the rotating shaft of the suture member 621 are located in the same transverse plane, and the other end of the first suture arm 625 is fixed with one end of the second suture arm 626. The second suture arm 626 is a bent arm, and the other end of the second suture arm 626 is separably connected with the suture needle 627. One end of the suture thread is tied to the suture needle 627, and the other end of the suture thread is tied to the suture needle of the suture member 622. When the rack 425 moves downwardly in the axial direction, the teeth on one side of the upper tooth segment 425a drive the gear 623 to rotate, the gear 623 rotates to drive the rotating shaft 624 to rotate in the first direction, and the rotating shaft 624 drives the first suture arm 625, the second suture arm 626 and the suture needle 627 to rotate in the first direction. When the suture needle 627 rotates in the first direction, an end of the suture thread is driven to rotate from the inside of the puncture core assembly to the outside of the puncture core assembly and pass through the human tissue on one side of the puncture opening. Teeth on the other side of the upper tooth segment 425a drive the suture member 622 to rotate in the second direction in the same way, so that the other end of the suture thread also rotates from the inside of the puncture core assembly to the outside of the puncture core assembly and passes through the human tissue on the other side of the puncture opening. The first direction is opposite to the second direction.

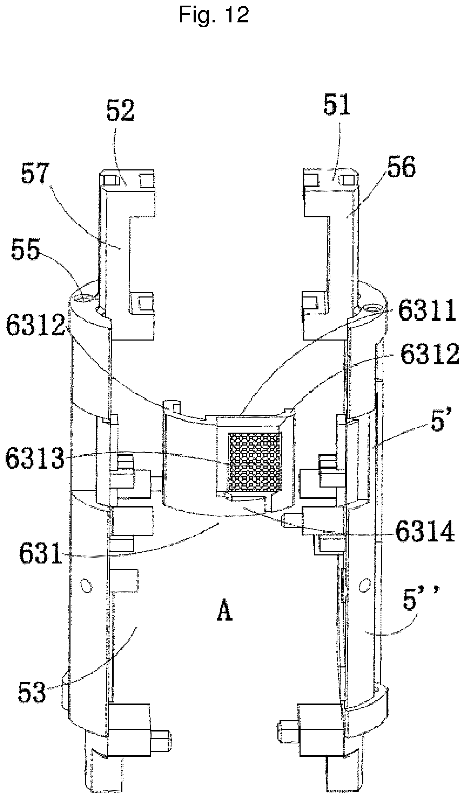

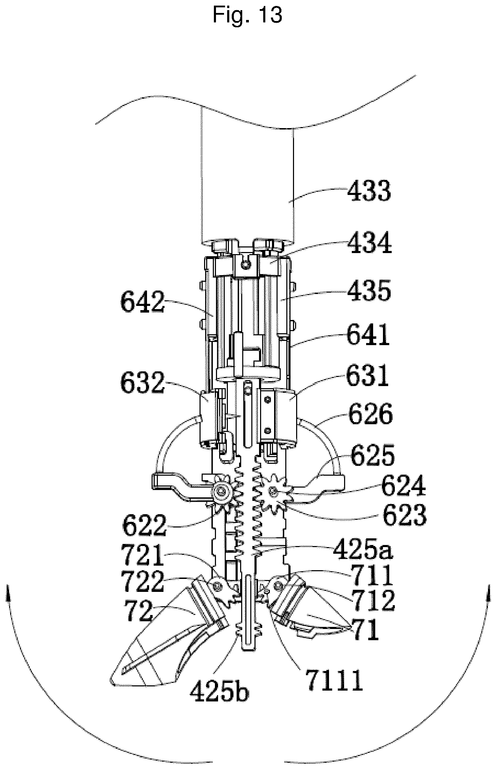

Referring to Fig. 12, the suture needle drives the end of the suture thread to pass through the human tissue around the puncture opening and is received by the receiving assembly 63. The receiving assembly 63 is disposed on the upper half part 5' of the supporting assembly 5. The receiving assembly 63 includes two receiving members 631 and 632 with the same structure. For simplicity of description, only the receiving member 631 is described below. The receiving member 631 includes a receiving part 6311, a holding part 6312 located on both sides of the receiving part 6311, and a receiving sheet 6313 located in the center of the receiving part 6311. The receiving part 6311 and the holding part 6312 are integrally formed, the holding part 6312 has a bent shape, the supporting assembly 5 is provided with a containing space matched with the holding part 6312 in shape, the first supporting member 51 fixes the holding part 6312 on one side, and the second supporting member 52 fixes the holding part 6312 on the other side, so that the receiving part 6311 is located above the containing space A, thus, the receiving part 6311 is located in the rotation path of the suture member 621. The first supporting member 51 is connected with the second supporting member 52 through two receiving members 631 and 632 with the same structure. The receiving sheet 6313 is disposed on the receiving part 6311. The receiving sheet 6313 is an elastic grid sheet or an elastic hollow sheet with a hollow structure. The grid of the elastic grid sheet or the hollow structure of the elastic hollow sheet is used to hold the suture needle 627. The rod wall pipe 3 is provided with a window 31, and the receiving sheet 6313 is located in the window 31. After passing through the human tissue on one side of the puncture opening, the suture needle 627 passes through the window 31 and is held by the receiving sheet 6313. The suture needle of the suture member 622 is synchronously held by the receiving sheet of the receiving member 632 in the same way, so as to receive the suture needle, as illustrated in Fig. 13.

In the present invention, the puncture opening is regarded as a hole, the upper part of the hole is in vitro, the lower part of the hole is in vivo, the cavity wall tissue of the human body is around the hole, the side surface of the puncture opening refers to the side wall of the hole, and the human tissue on both sides of the puncture opening refers to the cavity wall tissue of the human body around the hole. According to the present invention, the puncture opening is sutured from inside to outside, that is, the suture needle drives the suture thread to penetrate in from the lowest layer of tissue on both sides of the puncture opening (i.e. the fascia layer) and out from the side surface of the puncture opening, so that the fascia layer is well sutured. In the puncture core assembly provided by the present invention, the two ends of the suture thread are respectively tied to the suture needles of the suture members 621 and 622. Using the above suture mode, there will be a problem how to send the part of the suture thread except the two ends into the body cavity before suturing out the needle. In order to simplify the description, the part of the suture thread except the two ends is defined as a release part. In order to solve the above problems, the present invention makes the release part follow the lower half part of the puncture core assembly to pass through the puncture opening and enter the body cavity, specifically: the release part of the suture thread is contained in the puncture tip 7, so that the release part follows the puncture tip 7 to pass through the puncture opening and enter the body cavity. After the release part follows the puncture tip 7 to enter the puncture opening, it is necessary to release the suture thread during the suture operation to make the suture thread have sufficient length. In the present invention, the suture thread releasing action is executed by the puncture tip 7.

Referring to Figs. 13 and 14, the puncture tip 7 is conical. The puncture tip 7 has a tip inner cavity, which contains the release part of the suture thread. When the puncture tip 7 is opened, the release part of the suture thread expands to form a curve segment under the puncture tip 7 due to gravity and self elasticity to realize the function of releasing the suture thread. In order to enable the puncture tip 7 to be opened, the puncture tip 7 includes a first wall housing 71 and a second wall housing 72 which are separated from each other. The first wall housing 71 and the second wall housing 72 form a tip inner cavity after being closed. The first wall housing 71 and the second wall housing 72 are clamped with each other to make them close firmly. Specifically, a side surface of one of the first wall housing 71 and the second wall housing 72 is provided with a groove (not shown in the figures), and a side surface of the other is provided with a protrusion 73, which is held in the groove, so that the first wall housing 71 and the second wall housing 72 are clamped. The clamping makes that the first wall housing 71 and the second wall housing 72 are not separated when the puncture tip performs puncture, and the movement of the second transmission assembly 42 along the axial direction releases the clamping between the first wall housing 71 and the second wall housing 72 and separates the first wall housing 71 from the second wall housing 72. The first wall housing 71 and the second wall housing 72 are pivoted respectively to separate them from each other. The pivoting refers to the pivoting of the first wall housing 71 and/or the second wall housing 72 relative to the supporting assembly 5. In order to realize the pivoting of the first wall housing 71 relative to the supporting assembly 5, a toothed member 711 is disposed on the first wall housing 71, the toothed member 711 is sleeved on a first supporting shaft 712 through a first supporting hole, one end of the first supporting shaft 712 is fixed with the first supporting member 51, the other end is fixed with the second supporting member 52, therefore, the first supporting shaft 712 is fixed with the supporting assembly 5, the first wall housing 71 pivots around the first supporting shaft 712, the toothed member 721 is disposed on the second wall housing 72, the toothed member 722 is sleeved on a second supporting shaft 722 through a second supporting hole, one end of the second supporting shaft 722 is fixed with the first supporting member 51, the other end is fixed with the second supporting member 52, therefore, the second supporting shaft 722 is fixed with the supporting assembly 5, and the second wall housing 72 pivots around the second supporting shaft 722. In order to drive the first wall housing 71 to pivot around the first supporting shaft 711 and the second wall housing 72 to pivot around the second supporting shaft 722, the toothed members 711 and 721 are provided with several teeth, which are selectively engaged with the teeth of the lower tooth segment 425b of the rack 425, so that the relative pivoting between the first wall housing 71 and the second wall housing 72 and the rotation of the suture members 621 and 622 are synchronously driven by the rack 425. The relative pivoting includes pivoting of the first wall housing 71 and pivoting of the second wall housing 72, and pivoting of one of the first wall housing 71 and the second wall housing 72 and stationary remaining of the other. When only one of the first wall housing 71 and the second wall housing 72 needs to pivot, teeth are disposed on one side of the lower tooth segment 425b of the rack 425 and no teeth are disposed on the other side. In order to simplify the description, only a condition that both the first wall housing 71 and the second wall housing 72 are pivoted is described below: when the puncture tip 7 is in the initial state (i.e. not opened), the lower tooth segment 425b of the rack 425 is engaged with the toothed members 711 and 721. When the rack 425 moves downwardly, the teeth on one side of the upper tooth segment 425a drive the gear 623 to rotate in the first direction, the gear 623 rotates in the first direction to drive the rotating shaft 624, the first suture arm 625, the second suture arm 626 and the suture needle 627 to rotate in the first direction, and the teeth on one side of the lower tooth segment 425b drive the toothed member 711 to rotate in the first direction, and the toothed member 711 drives the first wall housing 71 to pivot in the first direction. At the same time, the teeth on the other side of the upper tooth segment 425a drive the suture member 622 to rotate in the second direction, the teeth on the other side of the lower tooth segment 425b drive the toothed member 721 to rotate in the second direction, and the toothed member 721 drives the second wall housing 72 to pivot in the second direction, so that both the first wall housing 71 and the second wall housing 72 pivot, the puncture tip 7 is opened, then the suture thread is released, and at this time, the puncture tip 7 is in the termination state. Because the present invention adopts a mode of suturing the puncture opening from inside to outside, the motion trajectory of the suture needle of each suture member is: rotating from the inside of the puncture core assembly to the outside of the puncture core assembly, and passing through the human tissue on one side of the puncture opening. This motion trajectory requires each suture member to rotate 180 degrees. Therefore, the rack 425 needs to move down enough length to make the suture members 621 and 622 rotate 180 degrees. However, the maximum pivoting angle of the first wall housing 71 and the second wall housing 72 and the downward movement length of the rack 425 are considered, when the first wall housing 71 and/or the second wall housing 72 pivot to the maximum angle, the rack 425 still moves downwardly, so that the toothed member 711 and the toothed member 721 are no longer engaged with the lower tooth segment 425b, at this time, it is necessary to keep the puncture tip 7 in the termination state, in order to be driven by the lower gear segment 425b and reset, in order to achieve the above purpose, a side surface of the toothed member 711 is protruded along the transverse direction to form a first limiting protruding block 7111, the inner surface of the first supporting member 51 or the second supporting member 52 has a first limiting clamping groove (not shown in the figures) for the first limiting protruding block 7111 to slide, the size of a part of the first limiting clamping groove is reduced, so it is in interference fit with the first limiting protruding block 7111, the part of the first limiting clamping groove corresponds to the position where the first limiting protruding block 7111 is located in the first limiting clamping groove when the first wall housing 71 pivots to the maximum angle, when the first wall housing 71 pivots along the first direction, the first limiting protruding block 711 rotates along the first direction with the toothed member 711, in the process of rotation, the first limiting protruding block 711 enters the first limiting clamping groove and slides in the first limiting clamping groove, when the first wall housing 71 pivots to the maximum angle, the first limiting protruding block 711 is clamped in the first limiting clamping groove, so that the toothed member 711 where the first limiting protruding block 7111 is located remains in the position, and the toothed member 711 remains in the position so that the first wall housing 71 remains open. And/or, an end face of the toothed member 721 is protruded outwardly along the transverse direction to form a second limiting protruding block (not labeled), the inner surface of the first supporting member 51 or the second supporting member 52 has a second limiting clamping groove (not shown in the figures) for the second limiting protruding block to side, the size of a part of the second limiting clamping groove is reduced, so that it is in interference fit with the second limiting protruding block, the part of the second limiting clamping groove corresponds to the position where the second limiting protruding block is located in the second limiting clamping groove when the second wall housing 72 pivots to the maximum angle, when the second wall housing 72 pivots along the second direction, the second limiting protruding block rotates along the second direction with the toothed member 711, in the process of rotation, the second limiting protruding block enters the second limiting clamping groove and slides in the second limiting clamping groove, when the second wall housing 72 is pivoted to the maximum angle, the second limiting protruding block is held in the second limiting clamping groove, so that the toothed member 721 where the second limiting protruding block is located remains in the position, the toothed member 721 remains in the position, so that the second wall housing 72 remains open, and the first wall housing 71 and/or the second wall housing 72 remain in the open state, so that the puncture tip 7 remains in the termination state. After the suture members 621 and 622 rotate 180 degrees and the suture needle is received by the receiving assembly 63 and fixed by the needle fixing assembly 64, the suture assembly 62 and the first wall housing 71 and/or the second wall housing 72 perform a reset action. The pressing cover 12 is pulled upwardly, so that the push member plate 1241 is abutted against the near end 424a of the transmission rod 424, the push member plate 1241 pulls the transmission rod 424, so that the second transmission assembly 42 where the transmission rod 424 is located moves upwardly, then the rack 425 moves upwardly, the teeth on one side of the upper tooth segment 425a of the rack 425 drive the gear 623 to rotate along the second direction, the gear 623 rotates along the second direction to drive the rotating shaft 624, the first suture arm 625, the second suture arm 626 and the suture needle 627 to rotate along the second direction, when the rack 425 moves upwardly for a certain distance, the teeth on one side of the lower tooth segment 425b encounter the toothed member 711 and are engaged with the toothed member 711, so as to drive the toothed member 711 to rotate along the second direction, and the toothed member 711 drives the first wall housing 71 to pivot along the second direction. At the same time, the teeth on the other side of the upper tooth segment 425a drive the suture member 622 to rotate along the first direction, the teeth on the other side of the lower tooth segment 425b drive the toothed member 721 to rotate along the first direction, and the toothed member 721 drives the second wall housing 72 to pivot along the first direction, so that the first wall housing 71 and the second wall housing 72 pivot towards each other until the first wall housing 71 and the second wall housing 72 are closed to form the puncture tip 7. At this time, the puncture tip 7 returns to the initial state, so that the suture assembly 62 performs the reset action synchronously with the first wall housing 71 and the second wall housing 72. It is to be noted that, the first wall housing 71 and the second wall housing 72 are two asymmetric structures. In this embodiment, the first wall housing 71 is smaller than the second wall housing 72, so that the puncture force is concentrated on the second wall housing 72, so as to avoid unexpected separation between the first wall housing 71 and the second wall housing 72 driven by the reaction force of human tissue on the puncture force concentration point during puncture. The suture assembly 62 and the puncture tip 7 share a rack to drive, which realizes the synchronous action of the two, meets the action logic relationship between the two, and saves the layout space of the puncture device.

Referring to Figs. 14 and 15, the third transmission assembly 43 sequentially includes an upper transmission ring 431, two symmetrical connecting rods 432, a third transmission pipe 433, a lower transmission ring 434 and two symmetrical booster arms 435 from top to bottom. The upper transmission ring 431, the third transmission pipe 433 and the lower transmission ring 434 are coaxial and have the same outer diameter. The upper transmission ring 431 is fixed with the near end of the connecting rod 432, the toggle arm 112 passes through a space between the two connecting rods 432 and is clamped with the first transmission pipe 411, and the far end of the connecting rod 432 is fixed with the near end of the third transmission pipe 433. The third transmission pipe 433 extends axially between the rod wall pipe 3 and the first transmission pipe 411. The far end of the third transmission pipe 433 is fixed with the lower transmission ring 434. The lower transmission ring 434 is fixed with the near end of the booster arm 435. The booster arm 435 is located between the first supporting member 51 and the second supporting member 52, so that the rotation of the booster arm 435 in the third transmission assembly 43 is prevented by the first supporting member 51 and the second supporting member 52, so that the rotation of the third transmission assembly 43 is prevented. Further, the outer surface of a first limiting part 56 and/or a second limiting part 57 has a convex point (not shown in the figures), the inner wall of the lower transmission ring 434 has a depression (not shown in the figures), and the convex point is contained in the depression, so as to prevent the lower transmission ring 434 from moving downwardly along the axial direction due to gravity, so that the third transmission assembly 43 where the lower transmission ring 434 is located remains stationary when it is not pressed down by the pressing cover 12.

When the pressing cover 12 is pressed from the second position and moves downwardly to the third position, the push member plate 1241 of the pressing cover 12 is abutted against the upper transmission ring 431 and pushes the third transmission assembly 43 to move downwardly, so that the lower transmission ring 434 moves against the resistance of the convex point, and the lower transmission ring 434 moves downwardly, so that the booster arm 435 moves downwardly along the axial direction between the first supporting member 51 and the second supporting member 52, thus, the needle fixing assembly 64 is driven to perform the needle fixing action. The end face where the containing hole 55 is located is the upper end face of the first supporting member 51 and the second supporting member 52.

It is to be noted that, since the pressing cover 12 needs to move downwardly enough to rotate each suture member 180 degrees, the needle fixing action is performed even after the suture needle is received by the receiving assembly 63. The above sufficient displacement is defined as h. When the pressing cover 12 is in the first position, the axial distance between the upper transmission ring 434 of the third transmission assembly 43 and the push member plate 1241 is greater than h, so that, when the pressing cover 12 is pressed and moved downwardly from the first position, each suture member rotates, and the third transmission assembly 43 remains fixed until each suture member has rotated 180 degrees after the pressing cover 12 moves downwardly for displacement h, at this time, the second transmission assembly 42 is fixed and does not drive the suture assembly 62 to act, the push member plate 1241 needs to move down for an idle stroke before it may abut against the upper transmission ring 434, so as to drive the needle fixing assembly 64 to perform the needle fixing action.

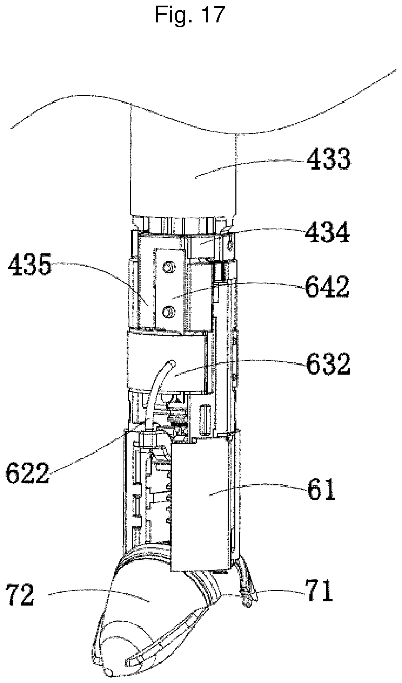

Referring to Figs. 13 and 15, the needle fixing assembly 64 is disposed above the needle receiving assembly 63. The needle fixing assembly 64 includes two symmetrical movable members 641 and 642. The movable member 641 is located above the receiving part 631 in the axial direction, and the movable member 642 is located above the receiving part 632 in the axial direction. In order to simplify the description, only the movable member 641 is introduced below. The near end of the movable member 641 is fixed on the outside of the booster arm 435, and the far end of the movable member 641 is an open end. The open end is realized by a limiting slit 641a, which is used to further hold the suture needle 627 held on the receiving sheet 6313. When the pressing cover 12 is in the second position, the pressing cover 12 is pressed, the pressing cover 12 moves downwardly to drive the third transmission assembly 43 to move downwardly, and the third transmission assembly 43 drives the movable member 641 to move downwardly. The movable member 641 moves downward to make the suture needle 627 roughly perpendicular to the limiting slit 641a to enter the limiting slit 641a until the suture needle 627 has been clamped into the near end of the limiting slit 641a, the suture needle 627 prevents the near end of the limiting slit 641a from continuously moving downwardly, so that both the movable member 641 and the third transmission assembly 43 can not move downwardly, so that the pressing cover 12 can not move downwardly, at this time, the pressing cover 12 is in the third position, as illustrated in Fig. 17. At this time, the far end of the movable member 641 is radially located between the receiving piece 631 and a blocking arm 6314, which is formed by extending from the holding part 6312 to the outside of the receiving part 6311. When the pressing cover 12 is in the third position, the pressing cover 12 is pulled, and the push member plate 1241 moves upwardly to abut against the near end 424a of the transmission rod 424. The push member plate 1241 moves upwardly, and the abutting distance with the near end 424a of the transmission rod 424 is equal to the distance that the pressing cover 12 moves from the third position to the second position. The push member plate 1241 pulls the transmission rod 424, and then pulls the whole second transmission assembly 42 to move upwardly. The second transmission assembly 42 drives the suture assembly 62 to perform reset. When the suture assembly 62 performs reset, the rack 425 moves upwardly along the axial direction, the teeth on one side of the upper tooth segment 425a drive the gear 623 to rotate, the gear 623 rotates to drive the rotating shaft 624 to rotate along the second direction, the rotating shaft 624 drives the first suture arm 625 and the second suture arm 626 to rotate along the second direction, the second suture arm 626 wants to drive the suture needle 627 to move while rotating, however, the movement of the suture needle 627 is prevented by the receiving sheet 6313 and the movable member 641, in this process, the suture needle 627 gives a reaction force to the receiving sheet 6313 and the movable member 641, the reaction force causes the movable member 641 to tilt up, but tilting is blocked by the inner surface of the blocking arm 6314, so that the movable member 641 maintains the holding of the suture needle 627, therefore, the suture needle 627 is held and separated from the second suture arm 626, after separation, the first suture arm 625 and the second suture arm 626 rotate from the outside of the puncture core assembly and return to the containing space A, and the suture needle 627 continues to be held in the receiving member 631, so as to realize the fixation of the suture needle 627. Since an end of the suture thread is tied to the suture needle 627, the end of the suture thread is also fixed in the receiving member 631 and the movable member 641. After the puncture core assembly is pulled out of the body, the end of the suture thread is also brought out of the body. By using the same method as described above, the suture needle of the suture member 622 is fixed by the movable member 642, so that the other end of the suture thread is also fixed in the receiving member 632 and the movable member 641, and can also be brought out of the body by the puncture core assembly. The movable member 641 maintains the fixation of the suture needle. The movable member 641 is matched with the blocking arm 6314.

In the present invention, erroneous execution of the needle withdrawing action performed by the suture assembly 62, the suture thread releasing action performed by the puncture tip 7 and the needle fixing action performed by the needle fixing assembly 64 can be avoided, and the avoidance of erroneous execution is realized by avoiding erroneous driving. Avoidance of erroneous driving of the needle withdrawing action and the suture thread releasing action is realized through that the turntable 11 prevents the pressing cover 12 from being wrongly pressed in the first position, and avoidance of erroneous driving of the needle fixing action is realized through that the turntable 11 prevents the pressing cover 12 from being wrongly pressed in the second position.

The fact that the turntable 11 prevents the pressing cover 12 from being wrongly pressed in the first position refers to that: also referring to Figs. 4 and 6, the turntable 11 also includes two symmetrical blocking wings (not labeled). Each blocking wing includes a blocking wing body and a blocking sheet 113. A strip-shaped blocking wing body (not labeled) is formed by protruding outwardly along the radial direction from the outer surface of the upper part of the housing 111, and the top of the blocking wing body extends upwardly along the axial direction beyond the upper end face of the housing 111 to form the blocking sheet 113. As illustrated in Fig. 4, when the turntable 11 is in the initial position, the blocking sheet 113 is abutted against the circumferential wall 123 of the pressing cover 12, so that the blocking sheet 113 prevents the pressing cover 12 from being wrongly pressed in the first position, so as to avoid the erroneous driving of the needle withdrawing action and the suture thread releasing action. As illustrated in Fig. 6, when the turntable 11 rotates from the initial position to the termination position, the turntable 11 rotates along the first circumferential direction until the blocking sheet 113 is staggered with the circumferential wall 123 and aligned with the notch 122. The blocking sheet 113 no longer blocks the downward movement of the pressing cover 12, so that the pressing cover 12 moves downwardly from the first position to the second position, so as to drive the suture assembly 62 to perform the needle withdrawing action and drive the puncture tip 7 to perform the suture thread releasing action.

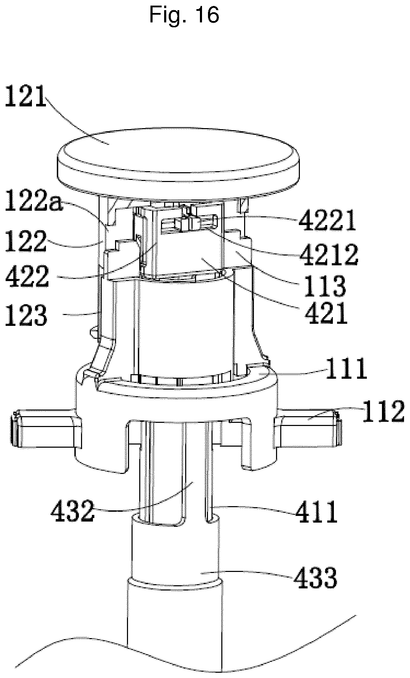

The fact that the turntable 11 prevents the pressing cover 12 from being wrongly pressed in the second position refers to that: also referring to Figs. 14 and 16, the tops of the two blocking sheets 113 are each provided with a first guide inclined plane 113a, the adjusting block 422 has a second guide inclined plane 422a extending between the bottom surface and the side surface, and the first guide inclined plane 113a is matched with the second guide inclined plane 422a. As illustrated in Fig. 14, when the pressing cover 12 is in the second position, the first guide inclined plane 113a is butted against the second guide inclined plane 422a, the first guide inclined plane 113a prevents the adjustment block 422 from moving downwardly, the adjustment block 422 further prevents the rib plate group 1221 from moving downwardly, and the rib plate group 1221 is prevented from moving downwardly, so that the pressing cover 12 is prevented from being wrongly pressed when in the second position, so as to avoid the erroneous driving of the needle fixing action. A pressing force on the pressing cover 12 is increased, so that the pressing of the rib plate group 1221 on the adjusting block 422 is increased. Since the pressing of the rib plate group 1221 on the adjusting block 422 is increased, the pressing of the second guide inclined plane 422a on the first guide inclined plane 113a is increased. Since the pressing of the second guide inclined plane 422a on the first guide inclined plane 113a is increased, the reaction force of the first guide inclined plane 113a on the second guide inclined plane 422a is increased. The reaction force is decomposed by the second guide inclined plane 422a to form a radial force and an axial force. The radial force is radially inward to make the two adjusting blocks 422 close to each other, so as to shrink the elastic member 423. The two adjusting blocks 422 close to each other, so that the adjusting block 422 moves from partially exposed to the adjusting frame 421 to fully contained in the adjusting frame 421, and the first guide inclined plane 113a no longer is abutted against the second guide inclined plane 422a, and the adjusting part changes from the first state to the second state. In the second state, the radial length of the adjusting part is reduced, and the adjusting block 422 no longer prevents the rib plate group 1221 from moving downwardly. The preset inclination angles of the first guide inclined plane 113a and the second guide inclined plane 422a make the adjusting block 422 move against the pressing exerted by the rib plate group 1221. The pressing cover 12 is continuously pressed. Since the radial length of the adjusting frame 421 is less than the radial distance between the two blocking sheets 113, the adjusting frame 421 is contained between the two blocking sheets 113. At this time, the two adjusting blocks 422 are subjected to the elastic force of the elastic member 423 to respectively abut against the inner surface of one of the two blocking sheets 113, so that the adjusting frame 421 is fixed between the two blocking sheets 113. Since the adjusting block no longer prevents the rib plate group 1221 from moving downwardly, when the pressing cover 12 continues to move downwardly, the blocking sheet 113 enters the slit 122a and moves relatively in the slit 122a. In the process, the pressing cover 12 is not in contact with the adjusting part, so that the second transmission assembly 42 where the adjusting part is located remains fixed without a driving force. At the same time, after the push member plate 1241 of the pressing cover 12 moves downwardly for an idle stroke, it touches the upper transmission ring 431, and then pushes the upper transmission ring 431, so as to move the whole third transmission assembly 43 downwardly, and the third transmission assembly 43 moves downwardly to drive the needle fixing assembly 64 to perform the needle fixing action. After the needle fixing action is performed, the pressing cover 12 is located in the third position, as illustrated in Fig. 15.

As a preferred one, in order to prevent the adjusting block 422 from being completely ejected out of the adjusting frame 421 by the elastic member 423 when the adjusting block 422 is in the static or moving process, and to avoid the problem of generating severe sloshing due to mutual friction between the adjusting block 422 and the adjusting frame 421 during the movement process of the adjusting block 422, as illustrated in Figs. 10 and 16, the side surface of the adjusting block 422 protrudes outwardly to form a guide protruding block 4221, and the adjusting frame 421 is provided with a guide groove 4212 for the radial movement of the guide protruding block 4221. When the adjusting block 422 is stationary, the guide protruding block 4221 is located in the guide groove 4212, and the guide groove 4212 limits the adjusting block 422 to prevent the adjusting block 422 from being completely ejected out of the adjusting frame 421. When the adjusting block 422 moves, the guide protruding block 4221 moves in the guide groove 4212, and the guide groove 4212 reduces the shaking amplitude when the adjusting block 422 moves. In order to further enhance the movement stability of the adjusting block 422, an anti-shaking protruding strip 4222 is disposed on the surface of the adjusting block 422. Due to the anti-shaking protruding strip 4222, the contact area between the adjusting block 422 and the adjusting frame 421 is reduced, thereby reducing the friction between them.

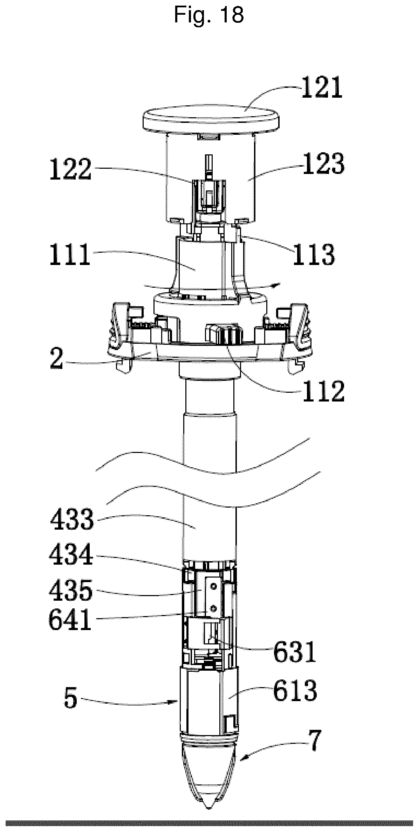

In conclusion, referring to Figs. 2-18, the puncture core assembly provided by the present invention has a part for driving, transmitting and executing a suture operation. During suture, a part of the puncture core assembly and a part of the cannula assembly are located outside the puncture opening, and a part is located in the puncture opening. When the suture operation is started, the toggle arm 112 of the turntable 11 is shifted along the first circumferential direction at first, and the toggle arm 112 rotates along the first circumferential direction to drive the first transmission assembly 41 to rotate. The first transmission assembly 41 drives the positioning blade of the positioning assembly 61 to rotate outwardly. After the positioning blade pivots outwardly, it protrudes out of the outer surface of the rod wall pipe 3, and the puncture core assembly is pulled upwardly, so that the upper end of the positioning blade butts against the fascia layer on both sides of the puncture opening to realize positioning. After the positioning blade is pivoted, the space where the suture assembly 62 is located in the containing space A is exposed, which is the suture channel 53. The pressing cover 12 is pressed downwardly, so that the pressing cover 12 moves downwardly. The pressing cover 12 moves downwardly to drive the second transmission assembly 42 to move downwardly. The upper tooth segment 425a of the rack 425 of the second transmission assembly 42 moves downwardly to drive the suture assembly 62 to rotate. The suture member 621 in the suture assembly 62 rotates along the first direction, and the suture member 622 rotates along the second direction to realize suturing out of the needle. The two suture needles rotate, and then enter the receiving assembly 63, the suture needle 627 of the suture member 621 is received by the receiving member 631, and the suture needle of the suture member 622 is received by the receiving member 632. The lower tooth segment 425b of the rack 425 moves downwardly to drive the relative pivoting of the first wall housing 71 and the second wall housing 72, so as to open the tip inner cavity. The suture thread releasing part in the tip inner cavity is separated and expanded from the inner cavity under the action of gravity and the elastic force of the suture thread itself to form a curve segment to realize the release of the suture thread. The pressing cover 12 is continuously pressed downwardly, so that the pressing cover 12 travels an idle stroke. At this time, the second transmission assembly 42 and the suture assembly 62 remain fixed, the push member 124 pushes the third transmission assembly 43 downwardly, the third transmission assembly 43 pushes the movable members 641 and 642 to move downwardly, the limiting slit 641a of the movable member 641 holds the suture needle 627, and the limiting slit of the movable member 642 holds the suture needle of the suture member 622, to achieve needle fixation. The pressing cover 12 is pulled upwardly, the pressing cover 12 moves upwardly to move the second transmission assembly 42 upwardly, the upper tooth segment 425a of the rack 425 of the second transmission assembly 42 rotates the suture assembly 62 in the opposite direction, the two second suture arms of the suture assembly 62 are separated from the respectively connected suture needles, and the first suture arm and the second suture arm rotate and return to the containing space A to realize the reset of the suture assembly 62. The lower tooth segment 425b of the rack 425 enables the first wall housing 71 and the second wall housing 72 to pivot relatively, and the first wall housing 71 and the second wall housing 72 are closed again to realize the reset of the puncture tip 7. In the process of upwardly pulling the pressing cover 12, the push member 124 is far away from the third transmission assembly 43, and the third transmission assembly 43 and the needle fixing assembly 64 remain stationary. After the pressing cover 12 is pulled to the first position, the toggle arm 112 is shifted along the second circumferential direction, the toggle arm 112 rotates along the second circumferential direction to drive the first transmission assembly 41 to rotate along the second circumferential direction, and the first transmission assembly 41 drives the positioning blade to pivot inwardly, and the positioning blade changes from the open state to the closed state to realize the reset of the positioning assembly 61, as illustrated in Fig. 18. Then, the whole puncture device is pulled upwardly, the two ends of the suture thread are brought out of the puncture opening by the puncture core assembly, the suture thread is cut to separate each end of the suture thread from the rest of the suture thread, and the suture thread is pulled out of the puncture opening to tighten the puncture opening and tie the suture thread to complete the suture operation.

The "suturable position" of the present invention refers to: when the puncture core assembly is in this position, the suture assembly 62 is driven, and the suture needle of the suture assembly 62 rotates to penetrate in from the tissue around the puncture opening, penetrate out from the side surface of the puncture opening and enter the needle receiving assembly 63.

The embodiments of the present invention have been shown or described above. However, it can be understood that the abovementioned embodiments are exemplary and should not be understood as limits to the present invention and those of ordinary skill in the art may make variations, modifications, replacements, transformations to the abovementioned embodiments within the scope of the present invention.

## Claims

1. A positioning mechanism for a puncture core assembly, comprising a positioning operating assembly and a positioning assembly, wherein the positioning assembly is driven by the positioning operating assembly to rotate with a first axial direction of the puncture core assembly as a central axis.

2. The positioning mechanism for the puncture core assembly according to claim 1, wherein the positioning operating assembly comprises a toggle member, which is applied with a force along a circumferential direction of the puncture core assembly, so as to drive the positioning operating assembly to rotate with a second axial direction of the puncture core assembly as a central axis, so as to drive the positioning assembly; and the first axial direction and the second axial direction are parallel and not coaxial.

3. The positioning mechanism for the puncture core assembly according to claim 2, wherein the second axial direction is a direction of a central axis of the puncture core assembly.

4. The positioning mechanism for the puncture core assembly according to claim 2 or 3, wherein the positioning mechanism also comprises a positioning transmission assembly, a near end of the positioning transmission assembly is fixedly connected with the toggle member, and a far end of the positioning transmission assembly is drivably connected with a near end of the positioning assembly.

5. The positioning mechanism for the puncture core assembly according to claim 4, wherein the far end of the positioning transmission assembly comprises a protruding part, the near end of the positioning assembly comprises a containing groove, and the protruding part is movably contained in the containing groove to form a driveable connection.

6. The positioning mechanism for the puncture core assembly according to claim 5, wherein the containing groove is a waist-shaped groove.

7. The positioning mechanism for the puncture core assembly according to claim 1, wherein the positioning assembly comprises a positioning blade, and a suture channel of the puncture core assembly is exposed after the positioning blade rotates.

8. The positioning mechanism for the puncture core assembly according to claim 5, wherein the far end of the positioning transmission assembly is a rotating ring, and the protruding part is formed by protruding from an upper surface or a lower surface of the rotating ring.

9. The positioning mechanism for the puncture core assembly according to claim 5, wherein the positioning assembly comprises a movement aiding arm, a pivot shaft and a positioning blade, the movement aiding arm extends horizontally from a near end of the pivot shaft, the movement aiding arm comprises the containing hole, and the positioning blade is located at a far end of the pivot shaft.

10. The positioning mechanism for the puncture core assembly according to claim 1, wherein the puncture core assembly also comprises a suture mechanism and a suture thread release mechanism.

11. The positioning mechanism for the puncture core assembly according to claim 1, wherein the positioning assembly has a closed state and an open state; and a conversion between the closed state and the open state is realized by a rotation of the positioning assembly with the first axial direction as the central axis.

12. The positioning mechanism for the puncture core assembly according to claim 11, wherein the positioning assembly comprises a positioning blade; in the closed state, the positioning blade is flush with an outer surface of the puncture core assembly; and in the open state, the positioning blade protrudes out of the outer surface of the puncture core assembly.

13. The positioning mechanism for the puncture core assembly according to claim 12, wherein the positioning blade has an upper surface which is perpendicular to a central axis of the puncture core assembly; and in the open state, the upper surface of the positioning blade is in surface contact with tissue.

14. A suture thread release mechanism for a puncture core assembly, comprising a first wall housing and a second wall housing, wherein the suture thread release mechanism has an initial state and a termination state, in the initial state, the first wall housing is closed with the second wall housing, in the termination state, the first wall housing is separated from the second wall housing, and when the first wall housing and the second wall housing are closed, the first wall housing and the second wall housing are surrounded by each other to form an inner cavity, which is used to contain a suture thread.

15. The suture thread release mechanism for the puncture core assembly according to claim 14, wherein a size of the first wall housing is different from a size of the second wall housing.

16. The suture thread release mechanism for the puncture core assembly according to claim 14, wherein the first wall housing is pivoted relative to the second wall housing to realize a conversion between the initial state and the termination state.

17. The suture thread release mechanism for the puncture core assembly according to claim 14, wherein one of the first wall housing and the second wall housing is provided with a toothed member, or, both the first wall housing and the second wall housing are provided with teeth members.

18. The suture thread release mechanism for the puncture core assembly according to claim 14, wherein the suture thread release mechanism also comprises a suture thread release operating assembly and a suture thread release transmission assembly, the suture thread release operating assembly is pressed or pulled to drive the suture thread release transmission assembly to move in a straight line, and the suture thread release transmission assembly drives a relative pivoting of the first wall housing and the second wall housing.

19. The suture thread release mechanism for the puncture core assembly according to claim 18, wherein the suture thread release operating assembly is butted against the suture thread release transmission assembly to drive the suture thread transmission assembly.

20. The suture thread release mechanism for the puncture core assembly according to claim 18, wherein the suture thread release transmission assembly comprises a rack.

21. The suture thread release mechanism for the puncture core assembly according to claim 20, wherein parts of two opposite side surfaces of the rack are provided with teeth, and the parts of the two side surfaces are symmetrical to each other; or, a part of a side surface of the rack is provided with teeth.

22. The suture thread release mechanism for the puncture core assembly according to claim 14, wherein in the initial state, the first wall housing and the second wall housing are closed to form a puncture tip, and the inner cavity is an inner cavity of the puncture tip.

23. The suture thread release mechanism for the puncture core assembly according to claim 18, wherein the puncture core assembly also comprises a suture mechanism, the suture mechanism comprises a suture operating assembly, a suture transmission assembly and a suture execution assembly, the suture operating assembly drives the suture execution assembly through the suture transmission assembly, the suture execution assembly comprises a suture needle, the suture transmission assembly and the suture thread release transmission assembly both comprise a same rack.

24. A suture needle fixing mechanism for a puncture core assembly, comprising a movable member, wherein the movable member comprises a slit extending from an end of the movable member, and the slit is used to hold a suture needle.

25. The suture needle fixing mechanism for the puncture core assembly as claimed in claim 24, wherein the suture needle fixing mechanism also comprises a suture needle fixing transmission assembly, the movable member is fixedly connected with the suture needle fixing transmission assembly, and the suture needle fixing transmission assembly drives the movable member to move in a straight line.

26. The suture needle fixing mechanism for the puncture core assembly as claimed in claim 25, wherein the suture needle fixing transmission assembly sequentially comprises an upper transmission ring, connecting rods, a transmission pipe, a lower transmission ring and two booster arms from top to bottom, the two booster arms are symmetrical disposed, there are two connecting rods which are symmetrically disposed with each other, the upper transmission ring is fixedly connected with near ends of the two connecting rods, far ends of the two connecting rods are fixedly connected with near end of the transmission pipe, far end of the transmission pipe is fixedly connected with the lower transmission ring, the lower transmission ring is fixedly connected with near ends of the two booster arms, there are two movable members, and the two movable members are fixedly connected with the two booster arms in one-to-one correspondence.

27. The suture needle fixing mechanism for the puncture core assembly as claimed in claim 25, wherein the suture needle fixing mechanism also comprises a suture needle fixing operating assembly, the suture needle fixing operating assembly is butted against the suture needle fixing transmission assembly after being pressed so as to drive the suture needle fixing transmission assembly to move in a straight line, and the suture needle fixing operating assembly is separated from the suture needle fixing transmission assembly after being pulled.

28. The suture needle fixing mechanism for the puncture core assembly as claimed in claim 27, wherein the puncture core assembly also comprises a suture mechanism and a suture thread release mechanism, the suture mechanism comprises a suture operating assembly, the suture thread release mechanism comprises a suture thread release operating assembly, and the suture needle fixing operating assembly, the suture operating assembly and the suture thread release operating assembly are the same operating assembly.

29. The suture needle fixing mechanism for the puncture core assembly as claimed in claim 28, wherein the same operating assembly comprises a pressing cover, which is pressed or pulled to move the same operating assembly downwardly or upwardly.

30. The suture needle fixing mechanism for the puncture core assembly as claimed in claim 24, wherein the suture needle fixing mechanism also comprises a blocking arm used to block the movable member holding the suture needle.

31. The suture needle fixing mechanism for the puncture core assembly as claimed in claim 24, wherein the suture needle fixing mechanism also comprises a receiving assembly, the receiving assembly comprises a receiving sheet, and the receiving sheet is an elastic grid sheet or an elastic hollow sheet used for holding the suture needle.

32. A suture mechanism for a puncture core assembly, comprising two suture members, wherein each suture member comprises a suture arm and a suture needle, the suture needle is separately connected to the suture arm, an end of a suture thread is fixed to the suture needle, and the two suture members are used for being driven to rotate to realize suturing out of a needle.

33. The suture mechanism for the puncture core assembly according to claim 32, wherein each suture member also comprises a gear, the suture mechanism also comprises a suture transmission assembly, the suture transmission assembly comprises a rack, and the gear is engaged with the rack.

34. The suture mechanism for the puncture core assembly according to claim 33, also comprising a suture operating assembly, wherein the suture operating assembly drives the suture transmission assembly to move in a straight line.

35. The suture mechanism for the puncture core assembly according to claim 33, wherein the puncture core assembly also comprises a suture thread release mechanism, and the suture thread release mechanism comprises a suture thread release transmission assembly, the suture transmission assembly and the suture thread release transmission assembly both comprise a same rack.

36. The suture mechanism for the puncture core assembly according to claim 34, wherein the puncture core assembly also comprises a suture needle fixing mechanism and a suture thread release mechanism, the suture needle fixing mechanism comprises a suture needle fixing operating assembly, the suture thread release mechanism comprises a suture thread release operating assembly, and the suture needle fixing operating assembly, the suture operating assembly and the suture thread release operating assembly are the same operating assembly.

37. The suture mechanism for the puncture core assembly according to claim 36, wherein the same operating assembly comprises a pressing cover, which is pressed or pulled to move the same operating assembly downwardly or upwardly.

38. A misoperation prevention mechanism, wherein a first part comprises a wall, a second part comprises a blocking part; the misoperation prevention mechanism comprises the wall and the blocking part; in an initial position, the blocking part is butted against the wall to prevent the first part from moving relative to the second part due to misoperation; the wall comprises a notch, when the first part is rotated and located in another position, the notch contains the blocking part, and then the first part is operated to move relative to the second part.

39. A misoperation prevention mechanism, wherein a first part comprises an adjusting block and a spring, one end of the spring is butted against the adjusting block, the other end of the spring is butted against other parts of the first part, except the adjusting block, an elastic force of the spring prevents the adjusting block from moving relative to the other parts; a second part comprises a blocking part; the blocking part has a first guide inclined plane, the adjusting block has a second guide inclined plane; in an initial position, the first guide inclined plane is butted against the second guide inclined plane, when a force of misoperation of the first part is not enough to overcome the elastic force of the spring, the blocking part is butted against the adjusting block to prevent the first part from moving relative to the second part due to misoperation; when an operating force is increased, the force of misoperation of the first part overcomes the elastic force of the spring to move the adjusting block towards the other part of the second part along the guide inclined plane, and when the adjusting block is separated from the blocking part, the first part is operated to move relative to the second part.

40. The misoperation prevention mechanism according to claim 39, wherein the first part also comprises another adjusting block, and the other end of the spring butts against the another adjusting block.

41. The misoperation prevention mechanism according to claim 39 or 40, wherein the first part comprises a guide groove, the adjusting block comprises a protruding part, and the protruding part is movably contained in the guide groove.

42. A puncture core assembly, comprising a suture mechanism, wherein the suture mechanism is the suture mechanism for the puncture core assembly according to any one of claims 32-37.

43. The puncture core assembly according to claim 42, wherein the puncture core assembly further comprises a positioning mechanism, wherein the positioning mechanism is the positioning mechanism for the puncture core assembly according to any one of claims 1-13.

44. The puncture core assembly according to claim 43, wherein the puncture core assembly further comprises a suture thread release mechanism, wherein the suture thread release mechanism is the suture thread release mechanism for the puncture core assembly according to any one of claims 14-23.

45. The puncture core assembly according to claim 43, wherein the puncture core assembly further comprises a suture needle fixing mechanism, wherein the suture needle fixing mechanism is the suture needle fixing mechanism for the puncture core assembly according to any one of claims 24-31.

46. The puncture core assembly according to claim 43, wherein the puncture core assembly further comprises a misoperation prevention mechanism, wherein the misoperation prevention mechanism is the misoperation prevention mechanism according to any one of claims 38-41.

47. A suturable puncture device, comprising a puncture core assembly and a cannula assembly, wherein the puncture core assembly is detachably sleeved on the cannula assembly, and the puncture core assembly is the puncture core assembly according to as claimed in claims 42-46.

48. A use method of a suturable puncture device, wherein the suturable puncture device comprises a puncture core assembly and a cannula assembly, the puncture core assembly is detachably sleeved on the cannula assembly, the puncture core assembly comprises a first operating assembly, a second operating assembly, a third operating assembly, a positioning blade, a rod wall pipe, a suture member, a suture thread release mechanism and a suture thread, the suture member comprises a suture needle, the use method comprises the following steps:
(1) operating the first operating assembly, so that the positioning blade rotates outwardly, and the positioning blade protrudes out of the outer surface of the rod wall pipe;
(2) upwardly pulling the puncture core assembly or the suturable puncture device, so that the positioning blade realizes positioning;
(3) operating the second operating assembly, so that the suture member rotates;
(4) operating the third operating assembly, so that the suture thread release mechanism is opened, and the suture thread contained in the suture thread release mechanism is released;
(5) rotating the suture member in place, and fixing the suture needle;
(6) further fixing the suture needle;
(7) operating the second operating assembly, so that the suture member returns;
(8) operating the third operating assembly, so that the suture thread release mechanism is closed;
(9) operating the first operating assembly, so that the positioning blade rotates inwardly; and
(10) upwardly puling the puncture core assembly or the suturable puncture device.

49. The use method of the suturable puncture device according to claim 48, wherein in the step (1), the operation is rotation.

50. The use method of the suturable puncture device according to claim 49, wherein the first operating assembly comprises a turntable, the step (1) also comprises: rotating the turntable, the turntable rotates along a first circumferential direction to drive a first transmission assembly to rotate, and the first transmission assembly drives the positioning blade to rotate outwardly.

51. The use method of the suturable puncture device according to claim 48, wherein the step (1) also comprises: exposing a suture channel after the positioning blade rotates outwardly.

52. The use method of the suturable puncture device according to claim 48, wherein in the step (3) and the step (4), all the operations are pressing operations.

53. The use method of the suturable puncture device according to claim 48, wherein in the step (3) and the step (4), the second operating assembly and the third operating assembly are the same operating assembly.

54. The use method of the suturable puncture device according to claim 48, wherein the puncture core assembly comprises a suture assembly, the suture assembly comprises the suture member, the suture member also comprises a gear; the second operating assembly comprises a pressing cover, the step (3) also comprises: pressing the pressing cover to enable the pressing cover to move downwardly, the pressing cover moves downwardly to drive a second transmission assembly to move downwardly, and a rack of the second transmission assembly moves downwardly to drive the gear to rotate so as to enable the suture member to rotate.

55. The use method of the suturable puncture device according to claim 48, wherein the third operating assembly comprises a pressing cover, the step (4) also comprises: pressing the pressing cover to enable the pressing cover to move downwardly, the pressing cover moves downwardly to drive a transmission assembly to move downwardly; and a rack of the transmission assembly moves downwardly to drive a toothed member of the suture thread release mechanism to rotate so as to open the suture thread release mechanism.

56. The use method of the suturable puncture device according to claim 54, wherein the third operating assembly comprises a pressing cover, the step (4) also comprises: pressing the pressing cover to enable the pressing cover to move downwardly, the pressing cover moves downwardly to drive a transmission assembly to move downwardly, a rack of the transmission assembly moves downwardly to drive a toothed member of the suture thread release mechanism to rotate so as to open the suture thread release mechanism, the second transmission assembly in the step (3) and the transmission assembly in the step (4) are a same transmission assembly, the rack in the step (3) and the rack in the step (4) are a same rack, and a rotation of the suture needle is synchronized with an opening of the suture thread release mechanism.

57. The use method of the suturable puncture device according to claim 48, wherein the step (5) also comprises: holding the suture needle by a receiving member to be fixed.

58. The use method of the suturable puncture device according to claim 48, wherein the step (6) also comprises: pressing the second operating assembly, the second operating assembly comprises a pressing cover, the pressing cover downwardly pushes a third transmission assembly, the third transmission assembly pushes a movable member to move downwardly, and a slit of the movable member holds the suture needle.

59. The use method of the suturable puncture device according to claim 48, wherein in the step (7) and the step (8), all the operations are pulling.

60. The use method of the suturable puncture device according to claim 48, wherein in the step (7) and the step (8), the second operating assembly and the third operating assembly are a same operating assembly.

61. The use method of the suturable puncture device according to claim 48, wherein the second operating assembly comprises a pressing cover, the step (7) also comprises: pulling the pressing cover to enable the pressing cover to move upwardly, the pressing cover moves upwardly to drive a second transmission assembly to move upwardly, and a rack of the second transmission assembly moves upwardly to drive a gear of the suture assembly to rotate so as to rotate and return the suture member.

62. The use method of the suturable puncture device according to claim 48, wherein the third operating assembly comprises a pressing cover, the step (8) also comprises: pulling the pressing cover to enable the pressing cover to move upwardly, the pressing cover moves upwardly to drive a transmission assembly to move upwardly, and a rack of the transmission assembly moves upwardly to drive a toothed member of the suture thread release mechanism to rotate so as to close the suture thread release mechanism.

63. The use method of the suturable puncture device according to claim 48, wherein in the step (9), operating the first operating assembly is rotating the first operating assembly.

64. The use method of the suturable puncture device according to claim 63, wherein the first operating assembly comprises a turntable, the step (9) also comprises: rotating the turntable, the turntable rotates along a second circumferential direction to drive a first transmission assembly to rotate, and the first transmission assembly drives the positioning blade to rotate inwardly.

65. The use method of the suturable puncture device according to claim 54, wherein in the step (3) and the step (4), the second operating assembly and the third operating assembly are a same operating assembly, the third operating assembly comprises a pressing cover, the step (4) also comprises: pressing the pressing cover to enable the pressing cover to move downwardly, the pressing cover moves downwardly to drive a transmission assembly to move downwardly, a rack of the transmission assembly moves downwardly to drive a toothed member of the suture thread release mechanism to rotate so as to open the suture thread release mechanism, the second transmission assembly in the step (3) and the transmission assembly in the step (4) are the same transmission assembly, the rack in the step (3) and the rack in the step (4) are the same rack, and the step (3) and the step (4) are executed synchronously.
